# EUROPÄISCHE PATENTANMELDUNG

(11) **EP 2 489 742 A1**
(43) Veröffentlichungstag der Anmeldung: **22.08.2012**
(21) Anmeldenummer: 12159119.2
(22) Anmeldetag: 09.10.2007
(51) Int. Cl.: C12Q 1/68

(54) **MicroRNA (MIRNA) zur Diagnose und Therapie von Herzerkrankungen**

(30) Priorität: 09.10.2006 EP 06090187
(62) Teilanmeldung aus: 07818845.5
(71) Anmelder: Julius-Maximilians-Universität Würzburg, 97070 Würzburg (DE)
(72) Erfinder: Thum, Thomas, 97082 Wuerzburg (DE); Bauersachs, Johann, 97074 Wuerzburg (DE)
(74) Vertreter: Carpmaels & Ransford

(57) **Zusammenfassung**

Die Erfindung betrifft MicroRNAs (miRNAs) zur Diagnose, Prophylaxe und/oder Therapie von Herzerkrankungen. Sie betrifft insbesondere SEQ ID NR: 1 bis SEQ ID NR: 29 zur Diagnose, Prophylaxe und/oder Therapie von Herzerkrankungen. Daneben betrifft die Erfindung die Verwendung dieser Sequenzen zur Herstellung eines Arzneimittels für Herzerkrankungen sowie zu deren Diagnose. Ferner ist ein Verfahren zur Diagnose einer Herzerkrankung, ein Kit und ein Expressionsvektor mit diesen Sequenzen, eine Zelle, die den Expressionsvektor enthält sowie ein Verfahren zum Modulieren einer Herzerkrankung und ein Verfahren zum Screenen einer pharmazeutisch aktiven Verbindung zur Behandlung und/oder Prophylaxe einer Herzerkrankung umfasst.

## Beschreibung

### Gebiet der Erfindung

Die Erfindung betrifft MicroRNAs (miRNAs) zur Diagnose, Prophylaxe und/oder Therapie von Herzerkrankungen.

Die Erfindung betrifft insbesondere SEQ ID NR: 1 bis SEQ ID NR: 29 zur Diagnose, Prophylaxe und/oder Therapie von Herzerkrankungen. Daneben betrifft die Erfindung die Verwendung dieser Sequenzen zur Herstellung eines Arzneimittels für Herzerkrankungen sowie zu deren Diagnose. Ferner ist ein Verfahren zur Diagnose einer Herzerkrankung, ein Kit und ein Expressionsvektor mit diesen Sequenzen, eine Zelle, die den Expressionsvektor enthält sowie ein Verfahren zum Modulieren einer Herzerkrankung und ein Verfahren zum Screenen einer pharmazeutisch aktiven Verbindung zur Behandlung und/oder Prophylaxe einer Herzerkrankung von der Erfindung umfasst.

### Hintergrund der Erfindung

### miRNAs

MicroRNAs (miRNAs) sind kleine nicht-kodierende RNA-Moleküle, welche posttranskriptionell die Genexpression durch Abbau der messenger-RNA regulieren können. Die Gesamtzahl verschiedener miRNAs wird auf ca. 300-500 geschätzt. Damit machen miRNAs ca. 1 % des menschlichen Genoms aus. miRNAs wurden in verschiedenen Spezies entdeckt und scheinen hoch konserviert zu sein.

Obwohl die Zielgene (Targetgene oder Targets) und somit die biologischen Funktionen der miRNAs bislang zum Großteil nicht identifiziert werden konnten, wird geschätzt dass miRNA bis zu 30 % der Gene des menschlichen Genoms regulieren.

Zunächst werden miRNA-Gene durch die RNA-Polymerase II in lange primäre miRNAs (pri-miRNA) transkribiert. Die weitere Prozessierung dieser pri-miRNAs erfolgt schrittweise und in verschiedenen Kompartimenten. Pri-miRNAs werden zunächst im Zellkern durch das RNase III-Enzym *Drosha* in ca. 70-80 Nukleotide umfassende Vorläufer miRNAs (precursor miRNAs; pre-miRNAs) umgewandelt. *Drosha* formiert sich mit dem RNA-bindenden Protein DGCR8 zu einem Mikroprozessor-Komplex. Pre-miRNA Haarnadelschleifen (hairpins) werden durch das Protein Exportin-5 und Ran-GTP als Kofaktor aus dem Zellkern herausbefördert. Im Zytoplasma wird die pre-miRNA durch das RNase II-Enzym *Dicer* in ca. 22 Nukleotide große Duplex-miRNAs prozessiert. *Dicer* interagiert hierbei mit dem doppelbandigen RNA-bindenden Protein TRBP. Die miRNA-Duplex-Moleküle werden anschließend entwunden, so dass reife miRNA entsteht. Diese reife miRNA wird anschließend in einen Ribonukleoprotein-Komplex inkorporiert (miRNP), welcher dem RNA-induzierten silcencing complex (RISC), dem Effektormolekül der interfering RNA (RNAi) sehr ähnlich ist (Hutvagner and Zamore, 2002).

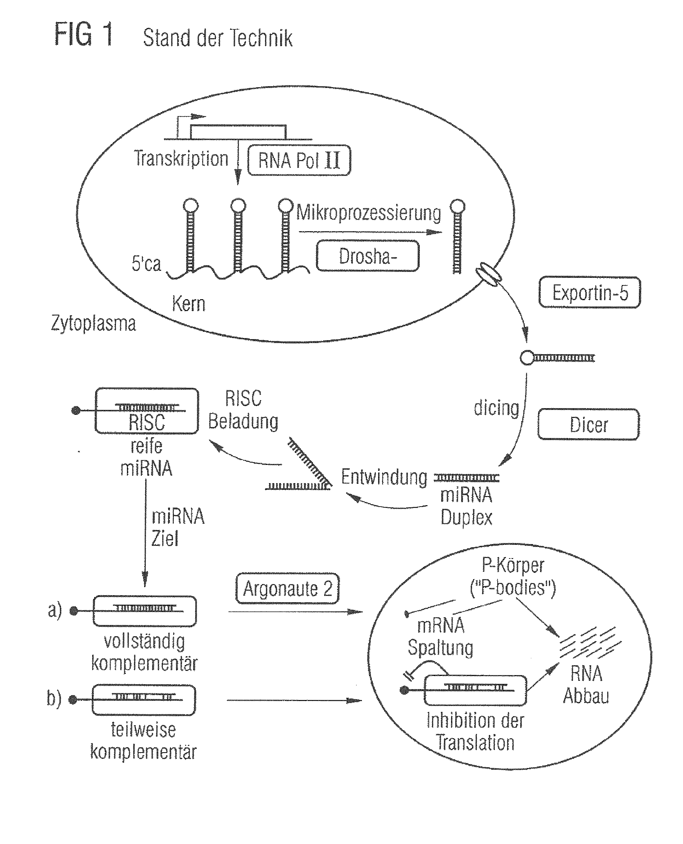

In dieser Form können miRNAs über zwei unterschiedliche Mechanismen zu einer Herabregulation des jeweiligen Zielgens führen: a) Translationale Inhibition oder b) Target-mRNA-Spaltung. Die Auswahl des Mechanismus hängt vom Grad der Komplementarität zwischen miRNA und dem Zielgen in Kombination mit einem sogenannten *Argonaute* Protein ab (Meister et al., 2005). Bei nahezu perfekter Komplementarität kommt es zu einer Spaltung des Zielgens mit anschließender RNA-Degradation; während es bei einer nur partiellen Komplementarität zu einer translationalen Inhibition kommt (Hutvagner and Zamore, 2002). Der genaue Mechanismus der translationalen Inhibition ist bislang nicht bekannt (vgl. Fig. 1, die den Stand der Technik darstellt).

Während bereits einige Mechanismen zur Steuerung von Differenzierungsprozessen durch miRNAs aufgeklärt werden konnten (Harfe, 2005; Hornstein et al., 2005), ist die Regulation physiologischer Funktionen durch miRNAs bislang weitgehend unbekannt.
Die große Anzahl an miRNAs und deren regulierte Targetgene prognostizieren eine wichtige Rolle von miRNA in der Entstehung und Progression verschiedenster Krankheiten. So ist beispielsweise die Mehrzahl der in unterschiedlichen Tumorproben identifizierten miRNAs herunterreguliert (Lu et al., 2005).

Über die Regulation der herzspezifischen Entwicklung liegt bislang in der Literatur eine Arbeit vor. Hier konnten Zhao und Mitarbeiter zeigen, dass bestimmte muskelspezifische miRNAs entscheidend an der Regulation der Herzentwicklung beteiligt sind (Zhao et al., 2005). So sind die miRNAs miR1-1 und miR1-2 besonders in kardialen Vorläuferzellen exprimiert. Ein Target von miR-1 ist der kardiale Transkriptionsfaktor Hand2, welcher wiederum die Expansion des Ventrikels während der Entwicklung kontrolliert. Sehr wahrscheinlich wirkt miR-1 einer zu schnellen und überschießenden Herzentwicklung entgegen (Zhao et al., 2005).

Eine weitere mögliche Rolle von miRNA in der Kontrolle von Umbauprozessen nach Myokardinfarkt oder anderen Herzerkrankungen, wie z.B. Hypertrophie oder Myokarditis scheint wahrscheinlich, jedoch sind Informationen hierüber bislang nicht verfügbar.

### Mögliche Bedeutung von miRNA bei Pathogenese und Heilungsprozessen verschiedener kardialer Erkrankungen

Die Defektheilung nach Myokardinfarkt mit Narbenbildung und konsekutiver reduzierter linksventrikulärer Pumpfunktion ist die häufigste Ursache der Herzinsuffizienz in den westlichen Industriestaaten (Massie and Shah, 1997). Die Hospitalisationsraten wegen Herzinsuffizienz haben sich in den europäischen Ländern in den letzten 10-15 Jahren nahezu verdoppelt. Die Prognose der Herzinsuffizienz ist ungünstig und ähnlich schlecht wie bei vielen malignen Tumoren. Bedingt durch die zunehmend höhere Lebenserwartung in der Bevölkerung werden Inzidenz und Prävalenz der Herzinsuffizienz weiter zunehmen. Die Herzinsuffizienz wird deshalb auch im neuen Jahrtausend eine der zentralen Herausforderungen für das Gesundheitswesen darstellen.

Der Begriff "kardiales Remodeling" (Pfeffer und Braunwald, 1990) beschreibt die Umbauprozesse des Herzens unter pathophysiologischen Bedingungen nach einem Myokardinfarkt oder bei anderen Erkrankungen (z.B. Myokarditiden), die zur Herzinsuffizienz führen können. Während es in der Frühphase nach Myokardinfarkt zur Nekrose des ischämischen Myokards mit konsekutiver Narbenbildung kommt, treten im Langzeitverlauf bei nicht direkt vom Infarkt betroffenem Myokard Veränderungen auf, die zunächst als Heilungs- und Anpassungsvorgänge aufzufassen sind. Die Ausdünnung und Dilatation der infarzierten Myokardwand kann früh (Tage bis Wochen) in der Postinfarktphase zur Infarktexpansion führen; das späte Remodeling (Monate bis Jahre) wird verursacht durch eine strukturelle Umgestaltung des überlebenden Myokards, welche durch Myozytenhypertrophie, interstitielle Fibrose, apoptotischen Kardiomyozytenverlust sowie Dilatation and Verformung der Herzkammer charakterisiert ist.

Die wesentlichen Charakteristika dieser strukturellen Veränderungen umfassen zelluläre Hypertrophie des verbleibenden Restmyokards, reduzierte Kapillarisierung sowie eine progrediente myokardiale Fibrose (Pfeffer and Braunwald, 1990). Zwar stellen diese initialen Umbauprozesse eine kompensatorische Antwort dar; letztendlich führen die Veränderungen aber zu einer progressiven linksventrikulären Dilatation und Einschränkung der linksventrikulären Pumpfunktion, welche durch eine hohe Mortalität gekennzeichnet sind.

Des Weiteren umfasst der Prozess des kardialen Remodelings molekulare, zelluläre und interstitielle Veränderungen. Betroffen sind nicht nur Kardiomyozyten (Phänotypänderung mit Hypertrophie und verminderter Kontraktilität, Nekrose, Apoptose), sondern auch insbesondere die Extrazellulärmatrix sowie eine Änderung des inflammatorischen Milieus. Das kardiale Remodeling wird beeinflusst durch hämodynamische Veränderungen, neurohormonale Aktivierung, inflammatorische Zytokine und eine Vielzahl weiterer Prozesse. Die chronische Herzinsuffizienz, wie auch der akute Myokardinfarkt, sind durch erhöhte Plasmaspiegel proinflammatorischer Zytokine (u.a. Tumor-Nekrosefaktor alpha, Interleukin-1-beta, -2 und -6) sowie deren lösliche Rezeptoren charakterisiert. Im insuffizienten Myokard werden inflammatorische Zytokine exprimiert. Neben der bekannten negativen inotropen Wirkung dieser Zytokine dürften längerfristig vor allem ausgeprägte Effekte auf den Myozyten-Phänotyp und die extrazelluläre Matrix bei chronischer Herzinsuffizienz von pathophysiologischer Bedeutung sein. Für eine aktuelle Übersicht über zelluläre und molekulare Mechanismen bei Herzinsuffizienz wird auf die weiterführende Literatur verwiesen (Hunter, 1999).

In klinischen Studien konnte eine signifikante Verbesserung der Prognose unter medikamentöser Therapie (Beta-Blocker, ACE-Inhibitoren, Aldosteron-Antagonisten) belegt werden. Trotzdem ist und bleibt die Herzinsuffizienz eine der häufigsten Todesursachen in den industrialisierten Ländern.

Die Suche und Entwicklung neuer Therapieformen zur Verbesserung kardialer Heilungsprozesse ist eine der größten Herausforderungen in der modernen Kardiologie geworden. Die Nutzung von miRNAs bietet neue Möglichkeiten für eine optimierte Therapie und/oder Diagnose von Herzerkrankungen.

Der vorliegenden Erfindung liegt deshalb die Aufgabe zugrunde, verschiedene miRNA Sequenzen anzugeben, die zur Diagnose, Prophylaxe und/oder Therapie von Herzerkrankungen geeignet sind.

### Zusammenfassung der Erfindung

Die Erfindung betrifft SEQ ID NR: 1 bis SEQ ID NR: 29 zur Diagnose, Prophylaxe und/oder Therapie von Herzerkrankungen. Daneben betrifft die Erfindung die Verwendung dieser Sequenzen zur Herstellung eines Arzneimittels für Herzerkrankungen sowie zu deren Diagnose. Ferner betrifft die Erfindung ein Verfahren zur Diagnose einer Herzerkrankung, einen Kit und einen Expressionsvektor mit diesen Sequenzen, eine Zelle, die den Expressionsvektor enthält sowie ein Verfahren zum Modulieren einer Herzerkrankung und ein Verfahren zum Screenen einer pharmazeutisch aktiven Verbindung zur Behandlung und/oder Prophylaxe einer Herzerkrankung.

Im Folgenden wird die Erfindung unter Bezugnahme auf die beigefügten Figuren unter Angabe von verschiedenen Ausführungsformen näher beschrieben.

### Kurzbeschreibung der Figuren

Fig. 1 stellt den Stand der Technik dar und zeigt die miRNA-Reifung und die Hemmungsprozesse der mRNA.
Fig. 2 zeigt isolierte miRNA und totale RNA durch Kapillarelektrophorese.
Fig. 3 zeigt eine hierarchische Clusteranalyse von deregulierten miRNAs - zu sehen sind die signifikanten Unterschiede in chronisch infarziertem Myokardgewebe (Infarkt; n=3) und Myokardgewebe von Schein-operierten Ratten (Sham; n=3).
Fig. 4 zeigt die Zunahme des linksventrikulären Gewichts nach der Verengung der Aorta (aortic banding) nach 3 Tagen (AB 3d) und 21 Tagen (AB 21d) gegenüber den Sham-Kontrollen (Sham).

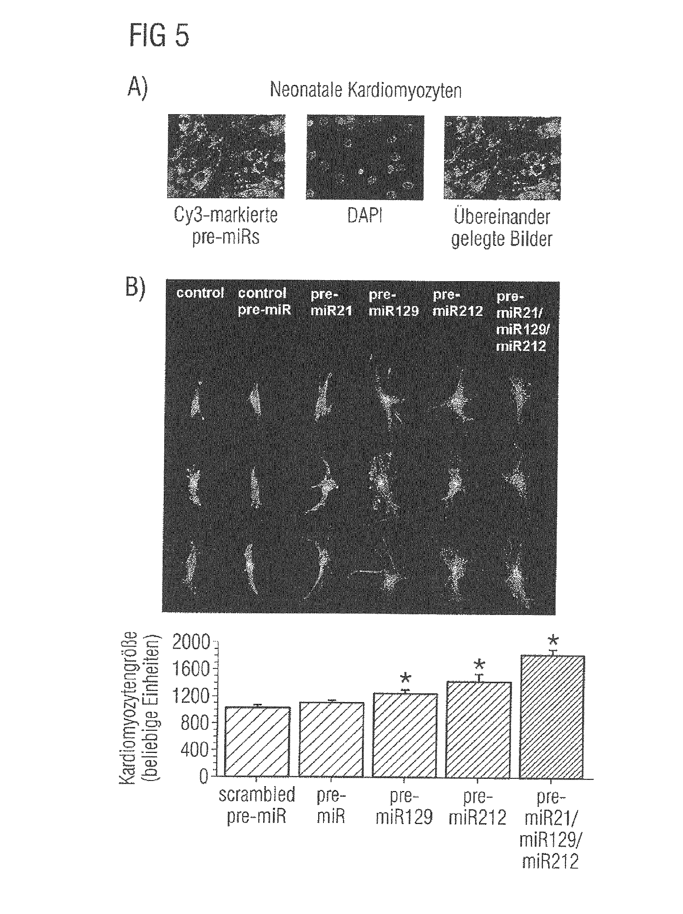

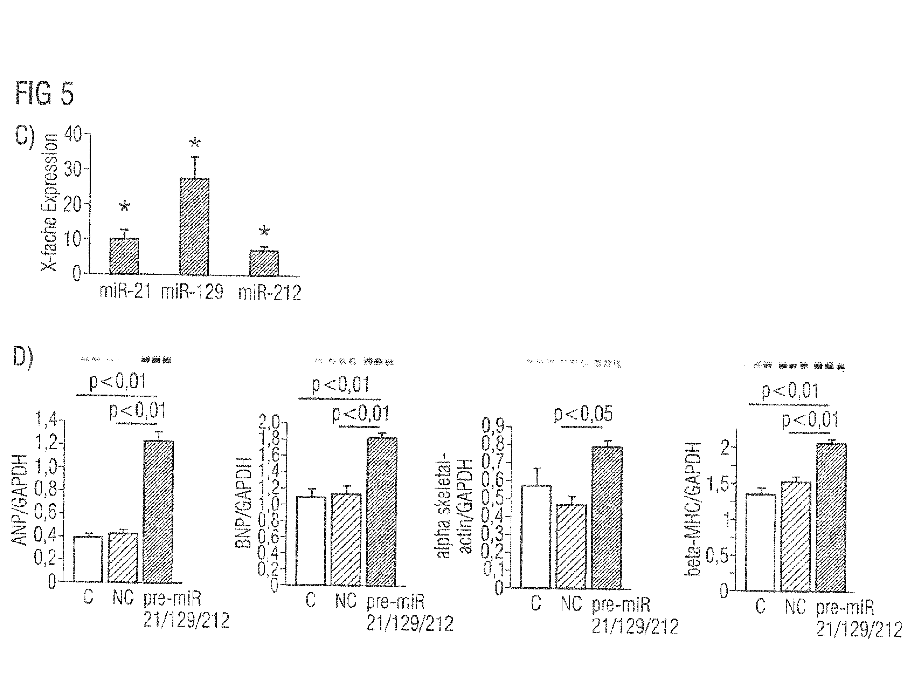

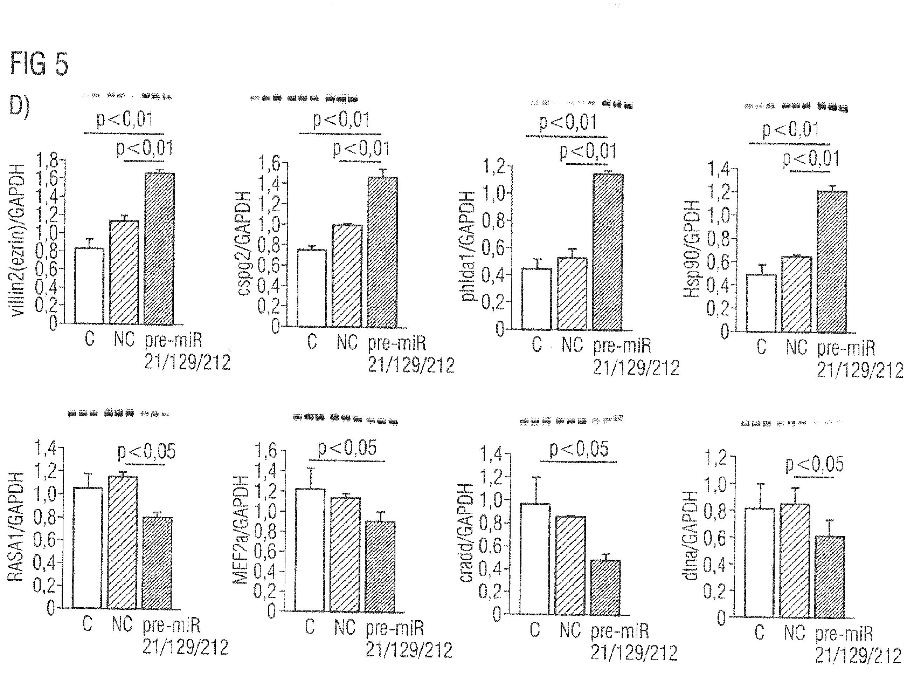

Fig. 5 zeigt miRNAs in kultivierte neonatale Kardiomyozyten (zu > 90%; restliche Zellen kardiale Fibroblasten und Endothelzellen) (A). Die Zellkerne wurden mit 4',6-Diamidino-2-phenylindol (DAPI) gefärbt. B) Zellgrößenzunahme unter miRNA-Behandlung. C) Verstärkte Expression einzelner miRNAs nach miRNA-Transfektion. D) Aktivierung fötaler Genprogramme nach miRNA Transfektion (ANP = atrial natriuretic peptide, BNP = brain natriuretic peptide; beta-MHC = beta-myosin heavy chain; cspg2 = chondroitin sulfate proteoglycan 2; phlda1 = pleckstrin homology-like domain, family A, member 1; HSP90 = heat shock protein 90; RASA1 = RAS p21 protein activator 1; MEF2a = myosin enhancer factor 2 alpha; cradd = CASP2 and RIPK1 domain containing adaptor with death domain; dtna = dystrobrevin alpha).
Fig. 6 zeigt miRNAs in kultivierte adulte Kardiomyozyten (zu > 90%; restliche Zellen kardiale Fibroblasten und Endothelzellen) (A), die Transfektionsspezifität (B), die verstärkte Expression einzelner miRNAs nach miRNA Transfektion (C), eine Zellgrößenzunahme unter miRNA Behandlung (D und F), und die Aktivierung fötaler Genprogramme nach miRNA-Transfektion (E). Die Zellkerne wurden mit 4',6-Diamidino-2-phenylindol (DAPI) gefärbt. (Die Abkürzungen sind wie oben angegeben).

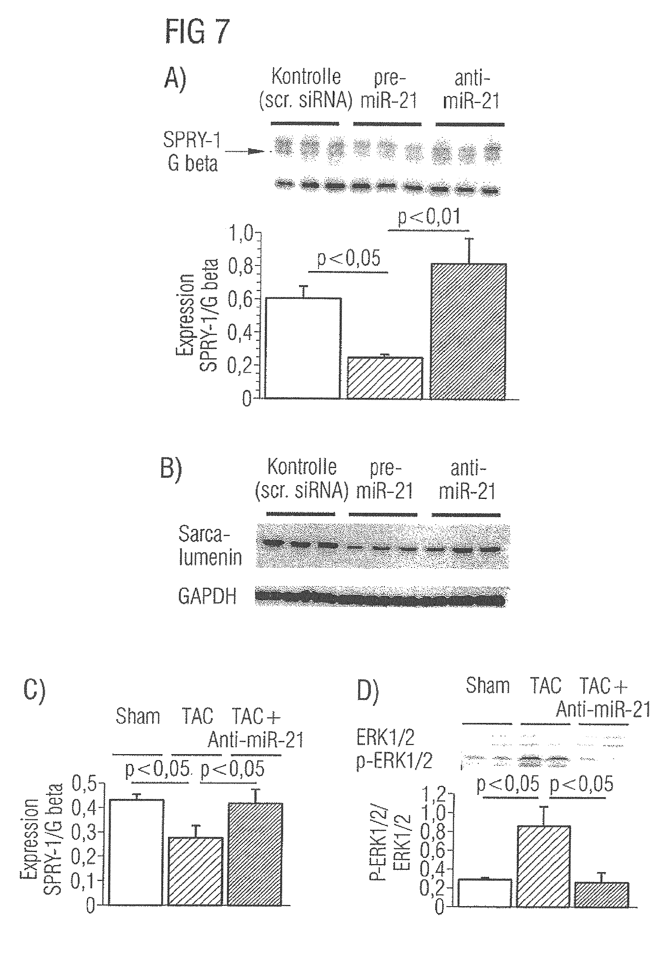

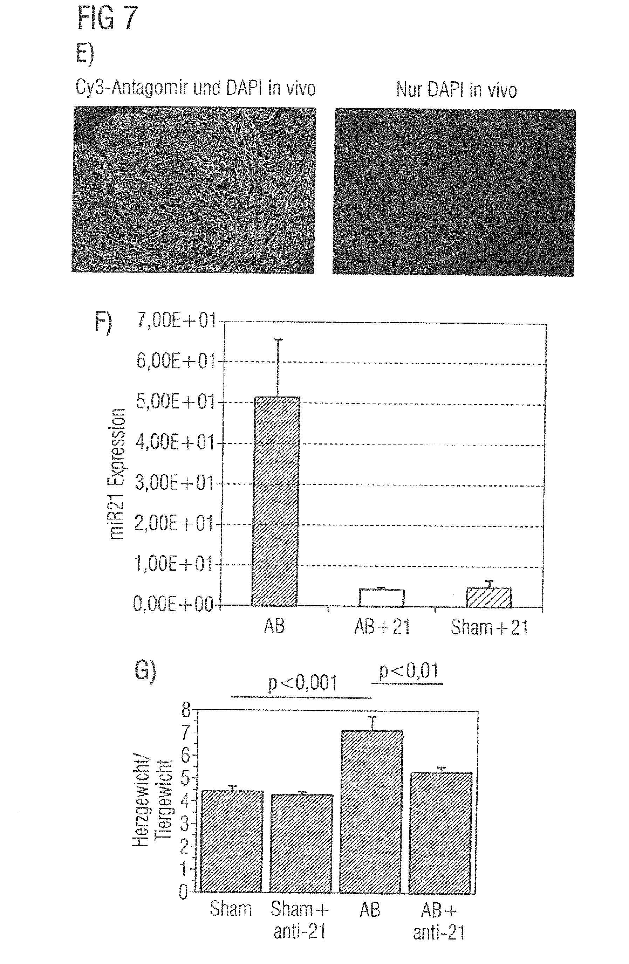

Fig. 7 zeigt die Repression von Sprouty-1 (A) und Sarcalumenin (B) nach pre-mir-21-Überexpression in kultivierten Kardiomyozyten (zu > 90%; restliche Zellen kardiale Fibroblasten und Endothelzellen). (C) und (D) zeigen die Repression von Sprouty-1 und die erhöhte ERK1/2-Phosphorylierung nach Druckbelastung des linken Ventrikels im Tiermodell (Aortic banding). (E) zeigt die Aufnahme von intravenös injiziertem Cy-3 markierten Antagomir® in das Herz. (F) zeigt eine Repression der kardialen miR-21-Expression nach Antagomir®-21 Behandlung. (G) zeigt eine Zunahme des Herzgewichts relativ zum Tiergewicht drei Wochen nach aortic banding, als auch die Verhinderung der Zunahme des Herzgewichts unter Antagomir®-21-Behandlung. (AB oder TAC = Aortic Banding Operation; anti-21 oder Anti-miR-21= Antagomir®-21 Behandlung).
Fig. 8A zeigt eine Größenzunahme der Kardiomyozyten nach aortic banding und die Normalisierung unter Antagomir®-21 Behandlung. (B) zeigt echokardiographische Analysen nach aortic banding mit und ohne Antagomir®-21 Behandlung. (Die Abkürzungen sind wie oben angegeben).

### Detaillierte Beschreibung der Erfindung und bevorzugte Ausführungsformen

Die vorliegende Erfindung betrifft miRNA-Sequenzen, insbesondere SEQ ID NR: 1 bis SEQ ID NR: 29 zur Diagnose, Prophylaxe und/oder Therapie von Herzerkrankungen. Die Sequenzen sind im Sequenzprotokoll angegeben und in Tabelle 2 zusammengestellt.

Ferner können die SEQ ID NR: 1 bis SEQ ID NR: 29 zur Herstellung eines Arzneimittels zur Prophylaxe und/oder Therapie von Herzerkrankungen verwendet werden.

Außerdem können die SEQ ID NR: 1 bis SEQ ID NR: 29 zur Diagnose von Herzerkrankungen verwendet werden.

In einer besonders bevorzugten Ausführungsform ist die Herzerkrankung ein Myokardinfarkt, eine Herzinsuffizienz, insbesondere eine chronische Herzinsuffizienz und/oder eine Herzhypertrophie.

Ein weiterer Gegenstand der Erfindung ist ein Verfahren zur Diagnose einer Herzerkrankung, wobei das Verfahren die Schritte umfasst:
(a) Bereitstellen einer Probe eines Individuums, das unter dem Verdacht einer Herzerkrankung steht;
(b) Messen der Expressionsmenge von mindestens einer Sequenz ausgewählt unter SEQ ID NR: 1 bis SEQ ID NR: 29 in der Probe;
wobei eine erhöhte oder verringerte Expressionsmenge mindestens einer Sequenz ausgewählt unter SEQ ID NR: 1 bis SEQ ID NR: 29 gegenüber einer Kontrollprobe eine Herzerkrankung oder eine Prävalenz bzw. Prädisposition für eine Herzerkrankung anzeigt.

Eine weitere Ausführungsform betrifft insbesondere die Verwendung der Sequenz SEQ ID NR: 5 (miR-134), SEQ ID NR: 10 (miR-212), SEQ ID NR: 11 (miR-214), SEQ ID NR: 14 (miR-21), SEQ ID NR: 20 (miR-182) und/oder SEQ ID NR: 24 (miR-290).

Besonders bevorzugt ist Insbesondere miR-21 (SEQ ID NR: 14). Diese miRNA ist in der humanen Herzinsuffizienz ca. 3,3-fach und früh nach Herzhypertrophie (aortic banding 3 Tage) ca. 3,0-fach bei der Maus hochreguliert.

Bevorzugt ist außerdem miR-212 (SEQ ID NR: 10). Diese miRNA ist in der humanen Herzinsuffizienz ca. 5,6-fach und nach chronischem Myokardinfarkt ca. 2,4-fach bei der Ratte hochreguliert.

Ferner ist miR-214 (SEQ ID NR: 11) bevorzugt. Diese miRNA ist leicht in der humanen Herzinsuffizienz ca. 1,2-fach und deutlich früh und spät nach Herzhypertrophie (aortic banding 3 und 21 Tage) ca. 1,5-fach, bzw. ca. 2,1-fach bei der Maus sowie nach chronischem Myokardinfarkt ca. 2,6-fach und nach pharmakologischer Herzhypertrophieinduktion *in vitro* ca. 1,5-fach hochreguliert.

Schließlich ist miR-134 (SEQ ID NR: 5) bevorzugt. Diese miRNA ist leicht in der humanen Herzinsuffizienz ca. 1,2-fach und deutlich spät nach Herzhypertrophie (aortic banding 21 Tage) ca. 1,7-fach bei der Maus sowie nach chronischem Myokardinfarkt (3,3-fach) in der Ratte hochreguliert.

Alle hochregulierten miRNAs können über miRNA-Inhibitoren *in vitro* und *in vivo* in ihrer Expression als Therapie normalisiert werden. Des Weiteren können sie als Diagnosemarker eingesetzt werden.

In einer alternativen Ausführungsform ist miR-182 (SEQ ID NR: 20) bevorzugt. Diese miRNA ist in der humanen Herzinsuffizienz (-1,3-fach) und deutlich früh und spät nach Herzhypertrophie (aortic banding 3 und 21 Tage) (-1,8-fach, bzw. -2,2-fach) bei der Maus sowie nach chronischem Myokardinfarkt (-4,0-fach) in der Ratte herunterreguliert.

Ferner ist miR-290 (SEQ ID NR: 24) bevorzugt. Diese miRNA ist in der humanen Herzinsuffizienz (-1,4-fach) und früh nach Herzhypertrophie (aortic banding 3 Tage) (-1,6-fach) bei der Maus sowie nach chronischem Myokardinfarkt (-1,4-fach) bei der Ratte herunterreguliert.

Diese reduzierten bzw. herunter regulierten miRNAs können über exogen hinzugegebene miRNAs normalisiert werden. Ferner können Medikamente entwickelt werden, welche die Expression der miRNAs normalisieren. Des Weiteren können sie, ggf. auch zusammen mit den hochregulierten miRNAs als Diagnosemarker verwendet werden.

Ein weiterer Gegenstand der Erfindung betrifft einen Kit umfassend mindestens eine Sequenz ausgewählt unter SEQ ID NR: 1 bis SEQ ID NR: 29 zur Diagnose von Herzerkrankungen.

Ferner ist ein Expressionsvektor umfassend mindestens eine Sequenz ausgewählt unter SEQ ID NR: 1 bis SEQ ID NR: 29 von der Erfindung umfasst.

In einer besonderen Ausführungsform gehört eine Zelle umfassend mindestens einen Expressionsvektor ebenfalls zur Erfindung.

Ein weiterer Aspekt der vorliegenden Erfindung betrifft ein Verfahren zum Modulieren einer Herzerkrankung umfassend das Modulieren der Expression oder Aktivität mindestens einer Sequenz, die ausgewählt ist unter SEQ ID NR: 1 bis SEQ ID NR: 29, in einer Zelle.

Noch ein Gegenstand der Erfindung betrifft ein Verfahren zum Screenen einer pharmazeutisch aktiven Verbindung zur Behandlung und/oder Prophylaxe einer Herzerkrankung, wobei das Verfahren die Schritte umfasst:
(a) Bereitstellen einer erfindungsgemäßen Zelle,
(b) In Kontakt bringen eines Kandidaten einer pharmazeutisch aktiven Verbindung mit der Zelle,
(c) Bestimmen der Wirkung des Kandidaten auf die Expression einer Sequenz, die ausgewählt ist unter SEQ ID NR: 1 bis SEQ ID NR: 29,
wobei eine Veränderung der Expression mindestens einer Sequenz von SEQ ID NR: 1 bis SEQ ID NR: 29 eine pharmazeutisch aktive Verbindung anzeigt.

Ferner sei angemerkt, dass sämtlichen Merkmalen, die in den Anmeldungsunterlagen und insbesondere in den abhängigen Ansprüchen genannt sind, trotz dem vorgenommenen formalen Rückbezug auf einen oder mehrere bestimmte Ansprüche, auch einzeln oder in beliebiger Kombination eigenständiger Schutz zukommt.

Die Erfindung wird in den folgenden Beispielen und Ergebnissen näher beschrieben, die nur exemplarisch angegeben sind und nicht einschränkend verstanden werden sollen.

### Beispiele und Ergebnisse

### 1. MicroRNA-Analysen nach Myokardinfarkt

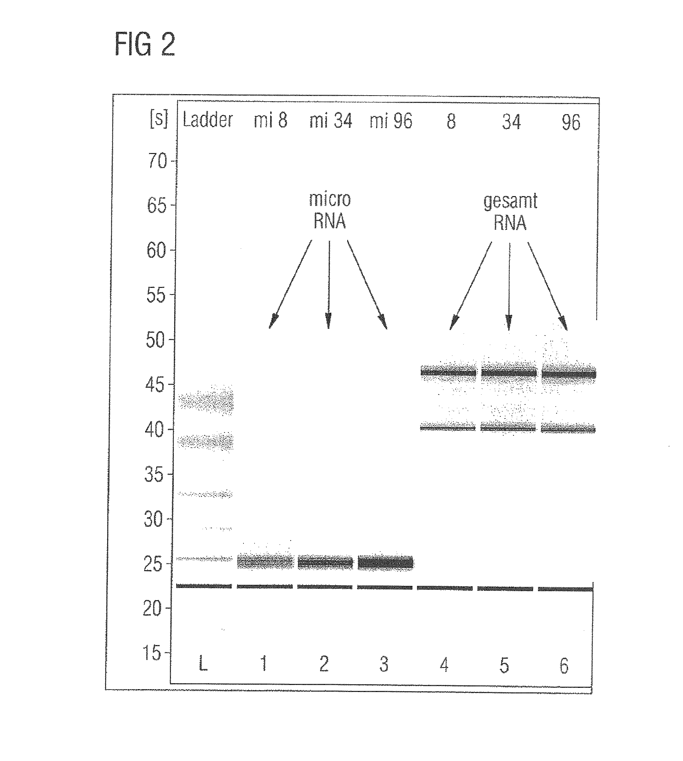

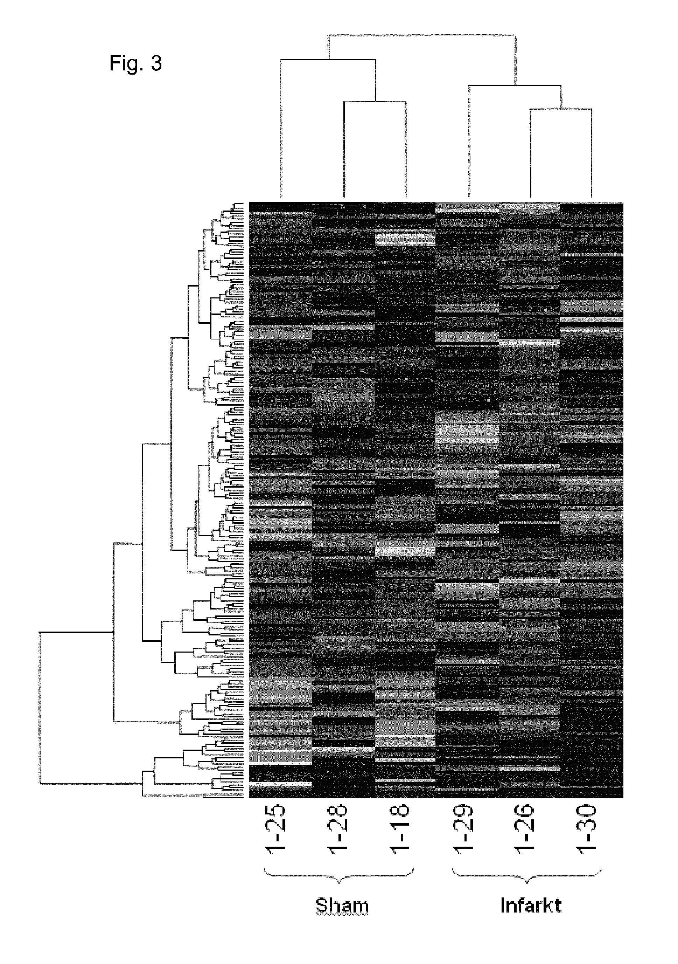

Im Labor wurden alle nötigen Verfahren zur Isolation und Microarray-basierten Detektion von >300 verschiedenen miRNAs aus Myokardgewebe etabliert. So konnte die miRNA-Isolation durch Hinzunahme eines weiteren Trennungsschrittes mittels Gelelektrophorese soweit optimiert werden, dass eine gute Markierung für die nachfolgende Hybridisierung und den Nachweis (Detektion) von miRNAs auf eigens gespotteten speziellen miRNA-Microarrays (in Kooperation mit der IZKF-Microarray Facility, Dr. S. Kneitz) möglich war (siehe Fig. 2 und Fig. 3). Durch Vergleich von gesundem mit chronisch infarziertem Myokard der Ratte konnten zahlreiche deregulierte miRNAs identifiziert werden, welche interessante Zielstrukturen für nachfolgende Forschungsansätze, bzw. interessante Diagnosemarker darstellen.

So konnte über Microarray-basierte Techniken durch die Erfinder bereits eine Anzahl im Herzen stark exprimierter miRNAs identifiziert werden. Zahlreiche differentiell exprimierte miRNAs im chronisch infarzierten Myokardgewebe deuten auf eine wichtige Rolle beim kardialen Remodeling hin.

Die folgenden miRNAs sind nach Myokardinfarkt induziert (> 1,2-fach der Schein (Sham)-operierten Tiere). Es sind Mittelwerte aus n=3 Tieren pro Gruppe angegeben:
miR211 (SEQ ID NR: 9), miR16-1, miR137, miR195, miR199a*-1 , miR199a-2, miR145, miR96, miR101-1, miR368, miR193, miR297-1, miR125b-1, miR1-2, let7f-1, miR295, miR199a-1, miR198, miR291-3p, miR99a, miR210, miR293, miR350, miR409, miR339, miR103-1, miR129-2, miR190, miR325, miR367, miR291-5p, let7d*, miR19a, miR369, miR300, miR199a*-2, miR98, miR221, miR292-3p, miR126, miR136, miR17-3p, miR128a (SEQ ID NR: 4), miR15b (SEQ ID NR: 1), miR151 (SEQ ID NR: 7), miR328 (SEQ ID NR: 12), miR126* (SEQ ID NR: 3), miR23a (SEQ ID NR: 2), miR212 (SEQ ID NR: 10), miR149 (SEQ ID NR: 6), miR206 (SEQ ID NR: 8), miR214 (SEQ ID NR:11), miR371 (SEQ ID NR: 13), miR134 (SEQ ID NR: 5).

Die folgenden miRNAs sind nach Myokardinfarkt reprimiert (< 80 % der Schein (Sham)-operierten Tiere). Es sind Mittelwerte aus n=3 Tieren pro Gruppe angegeben:
miR106b, miR182 (SEQ ID NR: 17), miR296 (SEQ ID NR: 18), miR122a (SEQ ID NR: 19), miR30a-3p (SEQ ID NR: 20), miR372, miR182*, miR298, miR9-1, miR155, miR99b, miR147, miR197, miR189, miR154, miR376b, miR219-1, miR202; miR370, miR341, miR27a, let7e, miR302c*; miR188, miR200b, miR215, miR142-3p, miR153-1, miR345, miR34a, miR21, miR148a, miR146, miR290, let7i, miR106a, miR217, let7g, miR183, miR200a, miR133a-1, miR7-1, miR22, miR9-1*, miR93, miR144, miR34c, miR220, miR141, miR130b.

Für ausgewählte miRNA Sequenzen ist die jeweilige SEQ ID NR: in Klammern angegeben.

Ein hierarchische Clusteranalyse nach Eisen (http://rana.lbl.gov/) zeigte ebenfalls die signifikanten Unterschiede in chronisch infarziertem Myokardgewebe (Infarkt; n=3) und Myokardgewebe von Schein-operierten Ratten (Sham, n=3) (siehe Fig. 3). Diese bioinformatorische Analysemethode sucht nach Unterschieden und Gemeinsamkeiten der verschiedenen untersuchten Gruppen.

Durch Suche in Datenbanken (http://microrna.sanger.ac.uk/) konnten bereits theoretische Zielgene der deregulierten miRNAs identifiziert werden. Eine Auswahl ist in Tabelle 1 angegeben.

**Tabelle 1**

| | |
|---|---|
| **Nach Myokardinfarkt herauf-regulierte miRNA und deren Zielgene, welche bei Myokardinfarkt/Herzinsuffizienz eine wichtige Rolle spielen** | |

| **miRNA** | **Zielgen** |
|---|---|
| **miR15b** | Carnitin-O-palmitoyltransferase II (Fettsäureoxidation) |
| (SEQ ID NR: 1) | Cyclin A2 |
| | G1/S-spezifisches Cyclin E1 |
| | Myosin-bindendes Protein C, Herz-Typ (Herz MyBP-C) |
| | Superoxide Dismutase [Cu-Zn] |
| | Fibroblasten Wachstumsfaktor 6 |
| | |
| **miR23a** | L-Lactat Dehydrogenase B Kette |
| (SEQ ID NR: 2) | Hypoxie-induzierbarer Faktor 1 alpha Inhibitor |
| | Retinoid X Rezeptor gamma |
| | Cyclin H |
| | Thioredoxin |
| | Cyclin-L1 (Cyclin-L) |
| | |
| **miRNA126*** | Vaskulärer Zelladhäsionsprotein 1 Vorläufer (V-CAM 1) |
| (SEQ ID NR: 3) | Vaskulärer endothelialer Wachstumsfaktor A Vorläufer (VEGF-A) |
| | Histon H2B |
| | |
| **miRNA128a** | Vaskulärer endothelialer Wachstumsfaktor C Vorläufer |
| (SEQ ID NR: 4) | Thioredoxin Reduktase 2 |
| | 6-Phosphofructokinase, Muskel-Typ |
| | Troponin I, Herzmuskel (Herz Troponin I) |
| | Myosin schwere Kette, Herzmuskel alpha Isoform (MyHC-alpha) |
| | Somatotropin Vorläufer (Wachstumshormon) |
| | Thioredoxin Reduktase 2 |
| | Metalloproteinase Inhibitor 1 Vorläufer (TIMP-1) |
| | |
| **miRNA134** | Calreticulin |
| (SEQ ID NR: 5) | Cytochrom C Oxidase Polypeptid Va, mitochondrialer Vorläufer |
| | Peroxiredoxin 1 (EC 1.11.1.15) (Thioredoxin Peroxidase 2) |
| | |
| **miRNA149** | Carnitin O-Acetyltransferase |
| (SEQ ID NR: 6) | Kalium Kanal spannungsvermittelte Subfamilie D Mitglied 3 |
| | Wachstumsarrest und durch DNA-Schädigung induzierte 45 beta |
| | Tropomyosin 1 alpha Kette (Alpha-Tropomyosin) |
| | |
| **miRNA151** (SEQ ID NR: 7) | Myosin-bindendes Protein C, Herz-Typ (Herz MyBP-C) (Herzmuskel Isoform) |
| | Calcium-aktivierte Kalium Kanal beta Untereinheit 4 |
| | Heparin-bindender Wachstumsfaktor Vorläufer (Saurer Fibroblasten Wachstumsfaktor) (aFGF). |
| | |
| **miRNA206** | Cytochrom b5 |
| (SEQ ID NR: 8) | Superoxid Dismutase [Cu-Zn] |
| | Cyclin H |
| | Angiopoietin-ähnliches 2 |
| | |
| **miRNA211** | Knochen morphogenetischer Protein 10 Vorläufer (BMP-1 0) |
| (SEQ ID NR: 9) | Mittlere Leitfähigkeit (intermediate conductance) Calcium-aktiviertes |
| | Kalium Kanal Protein 4 (SK4) (KCa4) |
| | Stickoxid Synthase, Gehirn m(EC 1.14.13.39) (NOS Typ I) (Neuro nales NOS) (N-NOS) |
| | Kalium Kanal spannungsvermitteltes Subfamilie H Mitglied 7 (Voltage-gated potassium channel subunit Kv11.3) |
| | Nestin |
| | Matrix Metalloproteinase-9 Vorläufer (EC 3.4.24.35) (MMP-9) |
| | Atriales natriuretischer Peptid Rezeptor B Vorläufer (ANP-B) |
| | Myogener Faktor 6 |
| | Vaskulärer Zelladhäsions Protein 1 Vorläufer (V-CAM 1) (CD106 Antigen) |
| | |
| **miRNA212** | Myosin-5B (Myosin Vb) (Myosin schwere Kette myr 6) |
| (SEQ ID NR: 10) | Calcium-aktivierte Kalium Kanal beta Untereinheit 4 |
| | Isocitrat Dehydrogenase [NAD] Untereinheit beta, mitochondrialer Vorläufer |
| | Transkriptionsfaktor E2F5 (E2F-5) |
| | Mitochondriales Carnitin/Acylcarnitin Trägerprotein |
| | Vaskulärer Zelladhäsionsprotein 1 Vorläufer (V-CAM 1) |
| | Ryanodin Rezeptor Typ 1 |
| | Metalloproteinase Inhibitor 1 Vorläufer (TIMP-1) |
| | Kalium Kanal spannungsvermitteltes Subfamilie A Mitglied 6 (Kv1.6) |
| | |
| **miRNA214** | Carnitin O-Acetyltransferase |
| (SEQ ID NR: 11) | Kreatin Kinase M-Typ |
| | Myosin-bindendes Protein H (MyBP-H) |
| | Metalloproteinase Inhibitor 4 Vorläufer (TIMP-4) |
| | Myosin XVIIIa |
| | Superoxid dismutase [Cu-Zn] |
| | Myosin leichtes Polypeptid 6 (Myosin leichte Kette Alkali 3) (Myosin leichte Kette 3) |
| | Herz Troponin C |
| | |
| **miRNA328** | Calcium-aktivierte Kalium Kanal beta Untereinheit 4 |
| (SEQ ID NR: 12) | Myosin-bindendes Protein H |
| | Calcium/Calmodulin-abhängige Proteinkinase Kinase 1 (EC 2.7.1.37) |
| | Natrium- und Chlor-abhängiger Kreatin Transporter 1 (CT1) (Crea tin Transporter 1) |
| | Kalium Kanal spannungsvermitteltes Subfamilie D Mitglied 3 (Kv4.3) |
| | IGF-II mRNA-bindendes Protein 1 |
| | Nestin |
| | Fructose-1,6-bisphosphatase 2 Myoglobin |
| | |
| **miRNA371** (SEQ ID NR: 13) | Somatotropin Vorläufer (Wachstumshormon) Kreatin Kinase B-Typ (EC 2.7.3.2) (Kreatin Kinase, B Kette) (B-CK) |

Einzelne stark regulierte miRNAs können über eine Heraufregulation (durch liposomale Transfektion der spezifischen miRNA) oder Herabregulation (durch liposomale Transfektion spezifischer miRNA-Inhibitoren (*anti-miRs*®, Ambion, UK oder *Antagomirs,* Alnylam, Kulmbach, Germany) der entsprechenden miRNA durch Applikation nach Myokardinfarkt moduliert werden. Alternativ können auch andere Transfektionsarten verwendet werden (z.B. viral oder durch Nukleofektion, bzw. Elektroporation) Durch die große Anzahl an Targetgenen einzelner miRNAs werden so ganze Gen-Netzwerke in ihrer Expression nach Myokardinfarkt wieder normalisiert und eine verbesserte Herzfunktion wiederhergestellt.

### 2. Methoden des miRNA-Expressionsscreens

### miRNA-Expressionsanalysen

Gefrorenes Herzgewebe oder kultivierte Kardiomyozyten wurden unter Verwendung von flüssigem Stickstoff zerkleinert. Aus ihnen wurde Gesamt-RNA einschließlich kleinen RNAs (small RNAs) isoliert (*mir*Vana™ miRNA Isolierungskit; Ambion, USA). Die Gesamt-RNA wurde getrennt isoliert und die gereinigten miRNAs unter Verwendung des FlashPAGE-Fraktionierungssystems (Ambion, USA) aufbereitet. Eine Kapillarelektrophorese (Bioanalyzer 2100; Agilent, Deutschland) wurde verwendet, um die Qualität der Gesamt-RNA und die Reinheit der miRNA zu bewerten.

MicroRNA, die aus 10 µg gesamt RNA erhalten wurde, wurde mit dem Farbstoff Cy3 (Molecular Probes, USA) unter Verwendung des *mir*Vana™ miRNA Markierungskits (Ambion, USA) nach den Angaben des Hersteller markiert. Jedes Target wurde in einem getrennten Array hybridisiert.

Microarrays mit bis zu 384 miRNAs (*mir*Vana miRNA Sondenset, Ambion) wurden im Labor der Erfinder auf SCHOTT Nexterion^{®} Slide E Microarray Objektträger jeweils 4-fach aufgespottet. Die Oligonukleotidsonden waren 42-46 nt lang und bestanden aus einem 18-24 nt Segment, das auf eine bestimmte bekannte miRNA, die vom Menschen, der Maus oder der Ratte stammt, abzielt.

Eine vollständige Auflistung der verwendeten Sonden einschließlich der Sequenzinformationen in dem Sondenset steht unter www.ambion.com/techlib/resources/- miRNA arrav/index.html zur Verfügung. Die Verarbeitung der Objektträger, die miRNA Reinigung, die Anreicherung und die Markierung wurde nach den Angaben in dem Handbuch von Ambion *mir*Vana™ (www.ambion.com/techlib/prot/) durchgeführt. Die Datenaufnahme wurde unter Verwendung der ScanAlyze Software (M. Eisen, LBNL, CA, USA) durchgeführt.

### Datenanalyse der Arrays

Die miRNA-Array-Datenanalysen wurden unter Verwendung des R-Pakets aus dem Bioconductor-Projekt (www.bioconductor.org) durchgeführt. Die resultierenden Signalintensitäten wurden durch Varianzstabilisierung normalisiert (Huber et al., 2002). Die Qualität aller Datensätze wurde getestet, und es wurde eine statistische Analyse durchgeführt, um differenziell exprimierte Gene unter Verwendung des Limma (Linear Models for Microarray Analysis) Pakets zu bewerten. Der Kern des Limma-Pakets ist eine Implementierung des empirischen Bayes linear modeling Ansatzes von Smyth and kann selbst für die stabilie Analyse von noch kleineren Probegrößen verwendet werden (Smyth, 2004).

### Verfahren zur Vorhersage des miRNA Targets (siehe Tabelle 1)

Die miRNA-Datenbank miRBase (http://microrna.sanger.ac.uk/) wurde verwendet um mögliche miRNA-Targets zu identifizieren. Der miRanda-Algorithmus wurde verwendet um alle zu Verfügung stehenden miRNA-Sequenzen eines gegebenen Genoms gegen die 3' UTR-Sequenzen dieses Genoms zu screenen. Der Algorithmus verwendet die dynamische Programmierung, um nach der maximalen lokalen Komplementarität zu suchen, die dem doppelsträngigen anti-parallelen Duplex entspricht. Ein positiver Wert (score) wird für komplementäre Basenpaarung angegeben, und ein negativer Wert (score) wird für Fehlpaarungen, Öffnungen und Erweiterungen der Transkriptions-/Replikationsgabel (gap-opening und gap-extension) angegeben. Die Werte, die von dem 5' Ende der miRNA abgeleitet waren, wurden mit einem Faktor multipliziert, um die Wichtigkeit einer perfekten Watson-Crick-Basenpaarung auszudrücken, die experimentell beobachtet wurde. Anschließend wurde eine Karlin-Altschul-Normalisierung durchgeführt (miRBase Targets Version 3.0; http://microrna.sanger.ac.uk/targets/v3/).

### MicroRNA-Analysen in humanen insuffizienten Herzen

Mit den bereits oben beschriebenen Methoden wurden miRNA-Expressionsprofile von n=4 humanen erkrankten Herzen und n=4 gesunden Herzen erstellt. Bei den erkrankten Herzen handelt es sich um linksventrikuläres Gewebe, das im Rahmen einer Herztransplantation aufgrund von dekompensierter Herzinsuffizienz entnommen wurde. Die deregulierten miRNAs (Herauf- sowie Herabregulation), die potenzielle Zielstrukturen für neue optimierte Therapien darstellen, sind im Folgenden angegeben:

Die folgenden miRNAs sind im menschlichen dekompensierten Herzen ("heart failure - Herzversagen") induziert (n=4 gesundes linksventrikuläres Gewebe; n=4 erkranktes dekompensiertes linksventrikuläres Gewebe):
hsa_miR_212 (SEQ ID NR:10), hsa_miR 21 (SEQ ID NR:14), hsa_miR_29b (SEQ ID NR:15), hsa_miR_129 (SEQ ID NR:16), mmu_miR_17_3p,, hsa_miR_210, hsa_miR_29a, hsa_miR_299_5p, hsa_miR_211, hsa_miR_423, hsa_miR_320, hsa_miR_200c, hsa_miR_1, hsa_miR_15a, hsa_miR_213 (SEQ ID NR:17), hsa_miR_126_AS, hsa_let_7c (SEQ ID NR:18), hsa_miR_26a, hsa_miR_106b (SEQ ID NR:19), hsa_miR_130a, hsa_miR_384, hsa_miR_424, hsa_let_7e, hsa_let_7g, mmu_miR_199b, hsa_miR_125a, hsa_miR_199b,, hsa_miR_28, hsa_miR_365, hsa_let_7d, hsa_miR_98, hsa_miR_335, hsa_let_7f, hsa_let_7b, hsa_miR_204, hsa_miR_372, hsa_miR_26b, mmu_miR_140_AS, hsa_miR_130b, mmu_miR_192, hsa_miR_34b, mmu_miR_129_3p, hsa_miR_132, hsa_miR_203, hsa_miR_181a, hsa_miR_127, mmu_miR_330, hsa_miR_15b, hsa_miR 199a, hsa_miR_516_3p, hsa_miR_101, hsa_miR_205, hsa_miR_151, hsa_miR_331, hsa_miR_32, hsa_miR_184, hsa_miR_489, hsa_miR_504, hsa_miR_31, hsa_miR_146a, hsa_miR_373, hsa_let_7a, hsa_miR_143, hsa_miR_511, hsa_miR_110b, hsa_miR_185, hsa_miR_136, rno_miR_349, hsa_miR_194, hsa_miR_145, hsa_miR_154, mmu_miR_151, hsa_miR_134 (SEQ ID NR: 5), mmu_miR_424, hsa_miR_181 c, mmu_miR_201, hsa_miR_508, hsa_miR_99b, hsa_miR_27b.

Die folgenden miRNAs sind im menschlichen dekompensierten Herzen ("heart failure - Herzversagen") reprimiert (n=4 gesundes linksventrikuläres Gewebe; n=4 erkranktes dekompensiertes linksventrikuläres Gewebe):
hsa_miR_139, hsa_miR_376a, hsa_miR_517a, hsa_miR_141, mmu_miR_380_3p, hsa_miR_30c, hsa_miR_520d, hsa_miR_519d,, mmu_miR_291_3p, hsa_miR_221, hsa_miR_520b, hsa_miR_522, hsa_miR_422b, hsa_miR_527, hsa_miR_17_3p, hsa_miR_507, hsa_miR_24, hsa_miR_523, hsa_miR_452_AS, hsa_miR_520a_AS, hsa_miR_135b, hsa_miR_182, hsa_miR_215, hsa_miR_518f_AS, hsa_miR_224, hsa_miR_339, mmu_miR_292_5p, mmu_miR_376b, hsa_miR_222, hsa_miR_96, mmu_miR_290 (SEQ ID NR: 24), hsa_miR_30a_5p (SEQ ID NR: 25), hsa_miR_425, hsa_miR_219 (SEQ ID NR: 26), hsa_miR_302b, hsa_miR_515_5p (SEQ ID NR: 27), hsa_miR_526b (SEQ ID NR: 28), hsa_miR_302b_AS, hsa_miR_107, hsa_miR_124a, hsa_miR_30b (SEQ ID NR: 29).

### Eine Überexpression bestimmter miRNAs führt zu zellulärer Hypertrophie und Aktivierung fötaler pathologischer Genprogramme in Kardiomyozyten

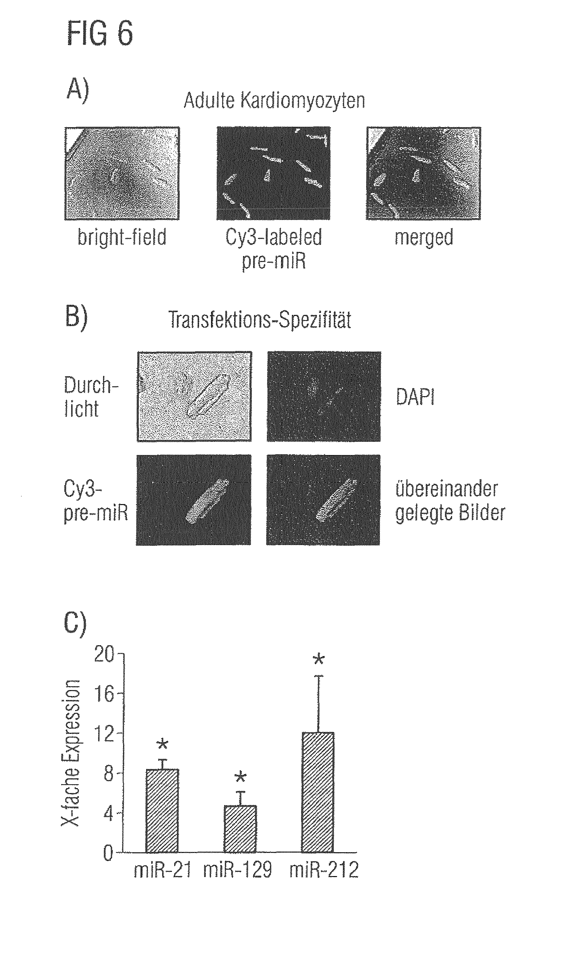

Kardiomyozyten wurden von neonatalen Ratten wie beschrieben isoliert und kultiviert (Burkard et al., 2005). Mehr als 95% der kultivierten Zellen zeigten eine Expression für das Kardiomyozyten-spezifische Aktinin, was auf eine sehr hohe Reinheit der Zellkultur schließen lässt. Fluoreszenz-Farbstoff (Cy3)-markierte scrambled-miR, -miR-21, -miR-129 und -miR-212-Moleküle (Cy3-pre-miR miRNAs, Ambion, USA, 100 nM, 48 h) wurden separat oder in Kombination mittels eines liposomalen Transfektionsverfahrens (Lipofectamine, Invitrogen, Germany) in kultivierte Kardiomyozyten transfiziert (Thum et al., 2007; Abb. 5A, B). 48 h nach der Transfektion wurden aus den Zellen Zell-Lysate wie beschrieben hergestellt (Thum et al., 2006). Adulte Kardiomyozyten wurden wie beschrieben isoliert und kultiviert (Thum et al., 2001). Adulte Kardiomyozyten wurden an ihrer typischen rechteckigen Morphologie identifiziert, und die Reinheit der Zellkultur an adulten Kardiomyozyten war sehr hoch (Abb. 6A). Adulte Kardiomyozyten wurden mit scrambled Cy3-markierter miRNA oder einem Set an miRNAs (miR-21, miR-129 and miR-212, 72 h, 25 nM) liposomal transfiziert (Lipofectamine, Invitrogen, Germany). Als ein Marker für die Zellgröße wurde die Oberfläche der kultivierten neonatalen (48 h nach Transfektion) und adulten (72 h nach Transfektion) Kardiomyozyten mit dem Bildanalyseprogramm AxioVison Rel. 4.4 (Carl Zeiss GmbH, Germany) bestimmt. Statistische Analysen wurden mittels one-way ANOVA gefolgt von mehreren Vergleichen mit dem "Fisher's protected least-significant difference" Test durchgeführt. Die statistische Analyse wurde mit dem Program Stat-View 5.0 (Abacus Concepts, Berkley, CA, USA) durchgeführt. Die statistische Signifikanz wurde für P<0.05 angenommen.

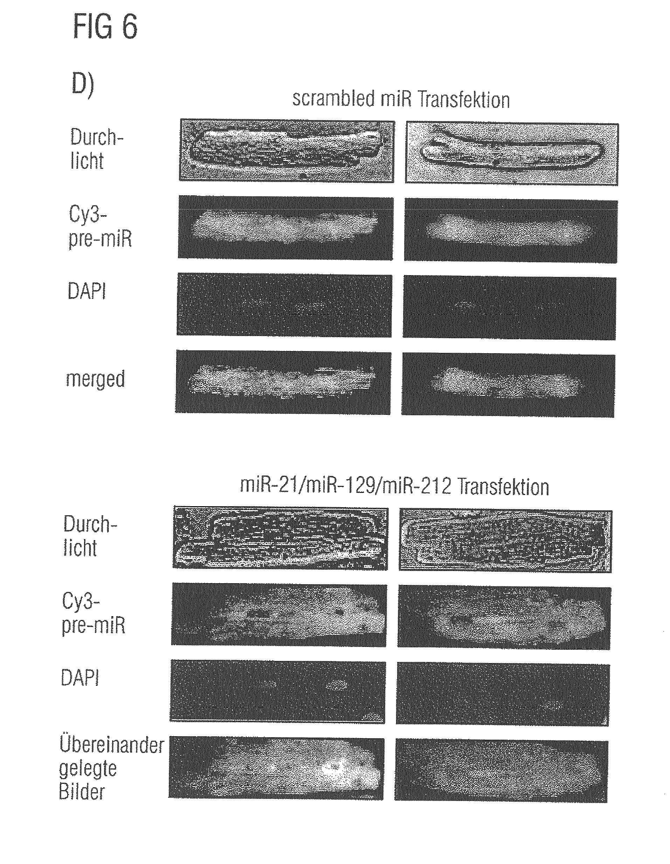

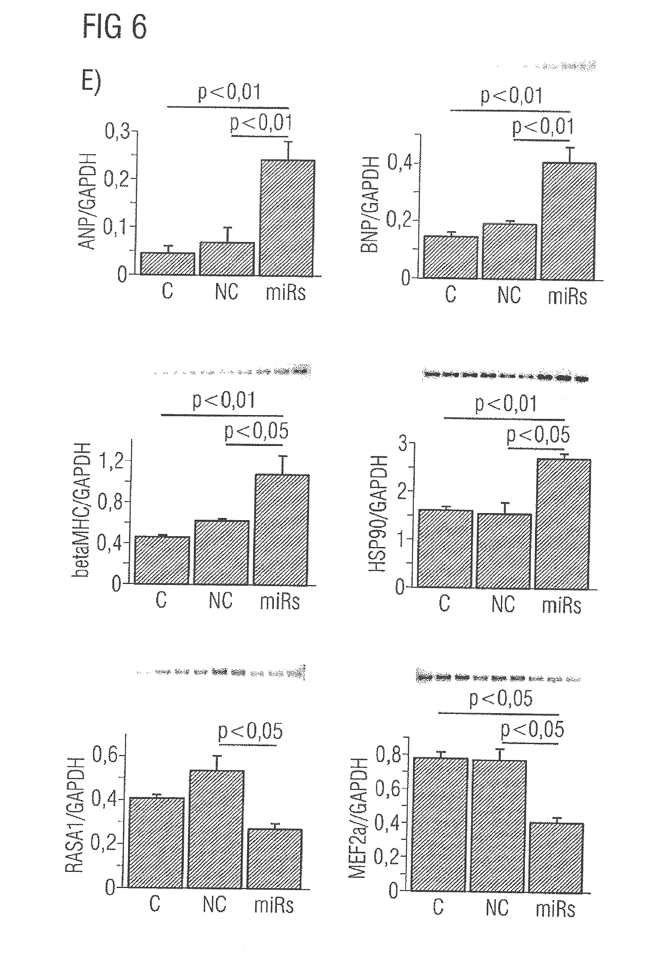

Um die Bedeutung einzelner deregulierter miRNAs für die Biologie von Kardiomyozyten zu analysieren, wurden drei in humanen insuffizienten Herzen hochregulierte miRNAs (miR-21, miR-129 and miR-212) in neonatalen und adulten Kardiomyozyten überexprimiert (siehe Abb. 5, 6). Die Transfektionseffizienz wurde mittels Nutzung Cy-3 markierter pre-miRNA-Moleküle untersucht und war sowohl in neonatalen als auch adulten Kardiomyozyten sehr hoch (siehe Abb. 5A, B; Abb. 6A; >85%). Zusätzlich wurde nach der Transfektion die Expression der einzelnen miRNAs mittles quantitativer miRNA Stammschleifen (stem loop) RT-PCR (Taq-Man® MicroRNA Assays, Applied Biosystems, USA) bestimmt. Die Transfektion führte in neonatalen und adulten Kardiomyozyten zu einer Heraufregulation der miRNAs miR-21, miR-129 und miR-212 (Abb. 5C, 6C). Nur vitale Kardiomyozyten konnten transfiziert werden (Abb. 6B). Während die Überexprimierung einzelner miRNAs zwar deutliche Effekte auf die zelluläre Morphologie, einschließlich Hypertrophie, ausübte, konnte der stärkste Effekt durch die gleichzeitige Überexpression aller drei miRNAs (miR-21, miR-129 and miR-212) erzielt werden (Abb. 5B, 6D). Eine weitere Folge der Überexpression dieser miRNAs war eine ReAktivierung fötaler Genprogramme, die regelmäßig während einer Herzerkrankung, einschließlich der Herzhypertrophie und Herzinsuffizienz, auftritt. So wurden die Gene *ANP, BNP, beta-MHC, alpha skeletal actin, villin2, cspg2, phlda1, hsp90, RASA1, MEF2a, cradd* und *dtna* durch die miRNA-Überexpression in ihrer Expression so moduliert, dass das Expressionsniveau auf dem krankhafter Herzen lag (Abb. 5D, 6E). Eine Transfektion adulter Kardiomyozyten mit diesem Set an miRNAs (miR-21, miR-129 und miR-212) resultierte ebenfalls in der Ausbildung einer zellulären Hypertrophie und Aktivierung fötaler Genprogramme (Abb. 6D, E, F). Die Transfektion von scrambled miRNAs führte weder zu einer zellulären Hypertrophie noch zu einer Aktivierung fötaler, pathologischer Genprogramme (Abb. 5, 6).

Die Daten identifizieren bestimmte miRNAs als wichtige Regulatoren für die ReAktivierung fötaler Genprogramme im insuffizienten humanen Herzen und liefern somit einen wichtigen Beitrag zur Erklärung der transkriptionellen Veränderungen bei der Herzinsuffizienz.

### MicroRNA-Analysen während der Entwicklung einer Myokardhypertrophie am Mausmodell in vivo und in vitro

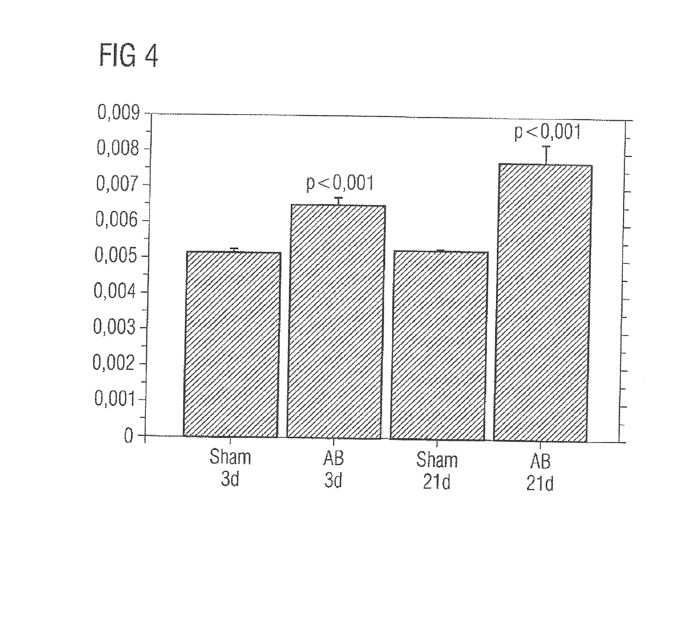

***In-vivo**:* Mittels eines operativen Eingriffs wurde der Durchmesser der Aorta an Mäusen stark verkleinert ("aortic banding"). Bei den Kontrollen ("Sham"-operierten Tieren) wurde ebenfalls der Thorax eröffnet und die Aorta dargestellt, aber keine Verengung der Aorta durchgeführt. Nach 3 und nach 21 Tagen wurde das linksventrikuläre Herzgewicht relativ zum Gesamtgewicht der jeweiligen Maus bestimmt (siehe Fig. 4) und anschließend die miRNA wie oben beschrieben isoliert. Danach wurden die miRNA-Expressionsprofile der Tiere mit verengter Aorta ("aortic banding-Tiere") im Vergleich zu den "Sham"-operierten Tiere nach 3 sowie 21 Tagen erstellt. Die deregulierten miRNAs (Herauf- sowie Herabregulation) stellen potenzielle Zielstrukturen für neue optimierte Therapien dar.

Die folgenden miRNAs sind nach 3 Tagen "aortic banding" induziert (n=4 Tieren pro Gruppe):
mmu_miR_106a, hsa_miR_21, hsa_miR_223, hsa_miR_512_3p, hsa_miR_377, mmu_miR_376b, hsa_miR_379, hsa_miR_197, hsa_miR_522, ambi_miR_7026, mmu_miR_295, hsa_miR_326, hsa_miR_214, ambi_miR_7027, mmu_miR_329, hsa_miR_488, mmu_miR_215, hsa_miR_521, rno_miR_20_AS, hsa_miR_491, hsa_miR_200a, hsa_miR_520a_AS, mmu_miR_376a, hsa_miR_301, hsa_miR_221, hsa_miR_510, hsa_miR_371, rno_miR_336, hsa_miR_520d, hsa_miR_20a, hsa_miR_380_3p, hsa_miR_323, mmu_miR_201, hsa_miR_365, hsa_miR_518a, mmu_miR_101b, hsa_miR_17_5p, hsa_miR_196b, hsa_miR_224, hsa_miR_217, hsa_miR_507, hsa_miR_520d_AS, hsa_miR_196a, hsa_miR_367, hsa_miR_369_3p, hsa_miR_99a, hsa_miR_141, ambi_miR_7039, hsa_miR_212, hsa_miR_524, hsa_miR_345, hsa_miR_220, mmu_miR_202, hsa_miR_106a, hsa_miR_107, hsa_miR_99b, hsa_miR_30b, hsa_miR_23b, hsa_miR_342, hsa_miR_518f, hsa_miR_339, hsa_miR_338, hsa_miR_302a.

Die folgenden miRNAs sind nach 3 Tagen "aortic banding" reprimiert (n=4 Tieren pro Gruppe):
hsa_let_7d, hsa_miR_30a_5p, hsa_miR_130a, ambi_miR_7089, hsa_miR_106b, hsa_miR_503, hsa_miR_485_5p, hsa_miR_496, hsa_miR_25, hsa_miR_493, ambi_miR_7081, hsa_miR_520h, hsa_miR_105, hsa_miR_490, hsa_miR_505, hsa_miR_452_AS, am-bi_miR_7085, hsa_miR_101, ambi_miR_7083, hsa_miR_216, am-bi_miR_7080, hsa_miR_500, rno_miR_346, ambi_miR_7066, am-bi_miR_7105, ambi_miR_7067, hsa_miR_497, hsa_miR_429, hsa_miR_302b_AS, hsa_miR_182, ambi_miR_7058, hsa_miR_499, hsa_miR_452, hsa_miR_511, hsa_miR_519b, hsa_miR_494, mmu_miR_140_AS, ambi_miR_7068_1, ambi_miR_7097, hsa_miR_337, hsa_miR_133a, ambi_miR_7059_1, hsa_miR_502, hsa_miR_202_AS, hsa_miR_432_AS, rno_miR_352, hsa_let_7b, mmu_miR_290, am-bi_miR_7084, rno_miR_349, hsa_let_7f, hsa_miR_504, hsa_miR_126, hsa_miR-1, mmu_miR_7b, mmu_miR_34b, hsa_miR_150, hsa_miR_30e_3p, hsa_miR_370, ambi_miR_7095, hsa_miR_190, hsa_miR_518c_AS, ambi_miR_7062, hsa_miR_375, mmu_miR_345, rno_miR_333, hsa_let_7a, hsa_miR_373, hsa_miR_495, hsa_miR_432, hsa_miR_492, mmu_miR_297, hsa_miR_515_3p, ambi_miR_7079, am-bi_miR_7036, hsa_miR_372, mmu_miR_346, mmu_miR_17_3p, mmu_miR_350, mmu_miR_129_3p, mmu_miR_330, ambi_miR_7101, hsa_miR_145, ambi_miR_7070, mmu_miR_341, hsa_miR_325, am-bi_miR_7076, hsa_miR_423, hsa_miR_135b, hsa_miR_422a, hsa_miR_34a, hsa_miR_34c, hsa_miR_142_5p, hsa_miR_30c.

Die folgenden miRNAs sind nach 21 Tagen "aortic banding" induziert (n=4 Tieren pro Gruppe):
hsa_miR_30a_5p, hsa_miR_106b, hsa_miR_25, hsa_miR_216, mmu_miR_297, hsa_miR_105, hsa_miR_101, hsa_miR_429, hsa_miR_512_3p, hsa_miR_138, hsa_miR_519b, mmu_let_7d_AS, hsa_miR_432, mmu_miR_341, hsa_miR_214, ambi_miR_7026, hsa_miR_222, hsa_miR_491, rno_miR_346, mmu_miR_291_3p, hsa_miR_524, rno_miR_327, ambi_miR_7039, mmu_miR_346, hsa_miR_367, mmu_miR_207, mmu_miR_351, mmu_miR_292_3p, hsa_miR_510, ambi_miR_7027, hsa_miR_379, hsa_miR_134, hsa_miR_525, hsa_miR_498, hsa_miR_198, hsa_miR_373, hsa_miR_512_5p, hsa_miR_136, hsa_miR_372, hsa_miR_518f, hsa_miR_514, hsa_miR_525_AS, hsa_miR_7, hsa_miR_506, hsa_miR_517a, ambi_miR_7085, mmu_miR_380_3p, hsa_miR_521, hsa_miR_130a, ambi_miR_7076, hsa_miR_296, hsa_miR_520d_AS, hsa_miR_346, hsa_miR_378, hsa_miR_324_3p, ambi_miR_7067, hsa_miR_520h, hsa_miR_206, hsa_miR_122a, hsa_miR_373_AS, mmu_miR_215, mmu_miR_409, hsa_miR_221, hsa_miR_330, am-bi_miR_7089, hsa_miR_129, mmu_miR_294, hsa_miR_196a, mmu_miR_376b, mmu_miR_383, ambi_miR_7074, hsa_miR_187, mmu_miR_300, hsa_miR_302d, hsa_miR_375, mmu_miR_292_5p, hsa_miR_518a, hsa_miR_92, ambi_miR_7066, hsa_miR_424, hsa_miR_518f_AS, hsa_miR_302c, hsa_miR_193a, hsa_miR_507, mmu_miR_429, rno_miR_20_AS, hsa_miR_200b, hsa_miR_339, hsa_miR_369_3p, mmu_miR_217, hsa_miR_517_AS, hsa_miR_527, hsa_miR_326, hsa_miR_377, mmu_miR_411, rno_miR_333, mmu_miR_344, mmu_miR_295, rno_miR_7_AS, ambi_miR_7100

Die folgenden miRNAs sind nach 21 Tagen "aortic banding" reprimiert (n=4 Tieren pro Gruppe):
hsa_miR_183, rno_miR_352, hsa_miR_133a, hsa_miR_10a, hsa_miR_203, hsa_miR_1, hsa_miR_202_AS, hsa_miR_450, hsa_miR_126, hsa_let_7a, hsa_miR_100, hsa_let_7b, hsa_miR_26b, hsa_let_7f, hsa_miR_26a, mmu_miR_337, hsa_miR_30c, hsa_miR_23b, hsa_miR_499, hsa_miR_29a, hsa_miR_23a, hsa_miR_10b, hsa_miR_24, hsa_let_7g, hsa_miR_30d, hsa_miR_30e_5p, mmu_miR_129_3p, hsa_miR_29c, hsa_miR_16, hsa_miR_145, hsa_miR_15b, hsa_miR_142_5p, hsa_miR_422b, hsa_miR_524_AS, hsa_miR_189, mmu_miR_106a, hsa_miR_27a, hsa_miR_422a, rno_miR_336, hsa_miR_150, hsa_miR_9_AS, ambi_miR_7029, hsa_miR_380_3p, hsa_miR_153, hsa_miR_490, hsa_miR_135b, hsa_miR_212, hsa_miR_125b, hsa_miR_22, hsa_miR_374, hsa_miR_195, rno_miR- 421, hsa_miR_200c, mmu_miR_151, ambi_miR_7059_1, hsa_let_7i, hsa_miR_140, hsa_miR_34a, hsa_miR_182, hsa_miR_501, hsa_miR_191, hsa_miR_342, hsa_miR_103, hsa_miR_27b, mmu_miR_140_AS, hsa_miR_204, hsa_miR_513, rno_miR_151_AS, hsa_miR_194, hsa_miR_125a, hsa_miR_345, hsa_miR_143, hsa_miR_15a, hsa_miR_520a, hsa_miR_36, hsa_miR_21, hsa_miR_219, hsa_miR_519e_AS, hsa_miR_497, hsa_miR_199a, hsa_miR_28, am-bi_miR_7103, ambi_miR_7105, mmu_miR_298, hsa_miR_197, hsa_miR_503, hsa_miR_34c, hsa_miR_526b, hsa_miR_485_5p, hsa_miR_508, hsa_miR_489, hsa_miR_526b_AS, hsa_miR_493, mmu_miR_199b, hsa_miR_188, hsa_miR_518d, mmu_miR_345, hsa_miR_181c, hsa_miR_99b, mmu_miR_322, hsa_miR_523, mmu_miR_291_5p, hsa_miR_148b, hsa_miR_526c, hsa_miR_302b_AS, hsa_miR_520b, hsa_miR_29b, hsa_miR_152, hsa_miR_149, hsa_miR_18a, hsa_miR_106a, hsa_miR_205, ambi_miR_7055, hsa_miR_186, hsa_miR_432_AS, hsa_miR_185, ambi_miR_7062, am-bi_miR_7080, hsa_let_7e, hsa_miR_192, mmu_miR_155, ambi_miR_7075, hsa_miR_30e_3p, hsa_miR_505, ambi_miR_7084, hsa_miR_494, am-bi_miR_7083, ambi_miR_7068_1, hsa_miR_17_3p, ambi_miR_7036, hsa_miR_518c, hsa_miR_34b, hsa_miR_148a, mmu_miR_330, mmu_miR_7b, hsa_miR_496, ambi_miR_7101, hsa_miR_199a_AS, hsa_miR_492, hsa_miR_324_5p, ambi_miR_7038_1, ambi_miR_7081, hsa_miR_412, hsa_miR_182 AS, hsa_miR_211, hsa_miR_151, hsa_miR_452, hsa_miR_337, hsa_miR_502, hsa_miR_193b, hsa_miR_376a, hsa_miR_299_5p, ambi_miR_7097, hsa_miR_519e, hsa_miR_19a, hsa_miR_488, hsa_miR_449, hsa_miR_320, hsa_miR_208, hsa_miR_518e, hsa_miR_410, hsa_miR_124a, hsa_miR_135a, hsa_miR_184, hsa_miR_181a, hsa_miR_516_3p, hsa_miR_328.

### In-vitro Induktion einer Hypertrophie an neonatalen Kardiomyozyten

Zunächst wurden neonatale Kardiomyozyten von Mäusen isoliert und kultiviert. Anschließend wurde eine 48 h Behandlung mit je 10 µM Phenylephrin und Isoproterenol durchgeführt, die zu einer zellulären Hypertrophie kultivierter Kardiomyozyten führte (Buitrago et al., 2005). In Folge wurden auch hier miRNA-Expressionsprofile behandelter und unbehandelter (nicht hypertrophierter) Kardiomyozyten erstellt. Die deregulierten miRNAs (Herauf- sowie Herabregulation), stellen potentielle Zielstrukturen für neue optimierte Therapien der Herzhypertrophie dar.

Die folgenden miRNAs sind nach 2 Tagen Behandlung (je 10 µM Phenylephrin und Isoproterenol) von kultivierten neonatalen Kardiomyozyten induziert (n=3 Versuche pro Gruppe):
hsa_miR_302c_AS, hsa_miR_101, hsa_miR_124a, hsa_miR_33, hsa_miR_220, hsa_miR_154, hsa_miR_99a, hsa_miR_340, hsa_miR_216, hsa_miR_323, hsa_miR_107, hsa_miR_302a, hsa_miR_141, hsa_miR_224, hsa_miR_98, hsa_miR_217, hsa_let_7c, hsa_miR_203, hsa_miR_213, mmu_miR_424, mmu_miR_106a, hsa_miR_302c, mmu_miR_217, hsa_miR_302b, hsa_miR_144, hsa_miR_32, hsa_miR_339, hsa_miR_205, hsa_miR_488, mmu_miR_291_3p, hsa_miR_17_3p, hsa_miR_338, hsa_miR_518a, hsa_miR_142_5p, mmu_miR_292_5p, hsa_miR_219, hsa_miR_302d, ambi_miR_7027, hsa_miR_384, hsa_miR_139, hsa_miR_137, hsa_miR_96, mmu_miR_292_3p, hsa_miR_136, hsa_miR_208, mmu_miR_17 3p, hsa_miR_18a, hsa_miR_215, hsa_miR_9, hsa_miR_142_3p, hsa_miR_181c, mmu_miR_140_AS, hsa_miR_130b.

Die folgenden miRNAs sind nach 2 Tagen Behandlung (je 10 µM Phenylephrin und Isoproterenol) von kultivierten neonatalen Kardiomyozyten reprimiert (n=3 Versuche pro Gruppe):
hsa_let_7d, hsa_miR_193b, ambi_miR_7080, hsa_miR_490, hsa_miR_497, ambi_miR_7081, hsa_miR_493, ambi_miR_7075, hsa_miR_502, hsa_miR_526c, hsa_miR_491, hsa_miR_199a_AS, am-bi_miR_7097, hsa_miR_485_5p, hsa_miR_501, hsa_miR_195, hsa_miR_505, hsa_miR_199a, mmu_miR_337, hsa_miR_30e_5p, hsa_miR_191, hsa_miR_489, hsa_miR_520e, mmu_miR_345, hsa_miR_189, hsa_miR_526b, hsa_miR_452, hsa_miR_432_AS, hsa_miR_30a_5p, hsa_miR_374, hsa_miR_496, ambi_miR_7068_1, hsa_miR_370, hsa_miR_34b, hsa_miR_499, mmu_miR_298, am-bi_miR_7076, mmu_miR_7b, hsa_miR_30b, hsa_miR_524_AS, hsa_miR_188, ambi_miR 7062, hsa_miR_130a, hsa_miR_382, rno_miR_346, hsa_miR_515_5p, hsa_miR_520a, hsa_miR_519c, hsa_miR_513, hsa_miR_526b_AS, hsa_miR_512_5p, ambi_miR_7059_1, hsa_miR_204, ambi_miR_7084, ambi_miR_7105, hsa_miR_23b, hsa_miR_500, hsa_miR_34c, hsa_miR_518c, hsa_miR_410, hsa_miR_492, ambi_miR_7095, hsa_miR_202_AS, hsa_miR_380_5p, am-bi_miR_7054, hsa_miR_25, hsa_miR_518b, hsa_miR_432, hsa_miR_30d, hsa_miR_449, hsa_miR_181a, hsa_miR_450, ambi_miR_7055, hsa_miR_29a, hsa_miR_19a, rno_miR_347, hsa_miR_373_AS, am-bi_miR_7083, hsa_miR_34a, hsa_miR_7, hsa_miR_24, rno_miR_421, mmu_miR_383, rno_miR_336, hsa_miR_181b, ambi_miR_7103, rno_miR_349, hsa_miR_361, mmu_miR_429, mmu_miR_384, hsa_miR_100, hsa_miR_519e_AS, mmu_miR_409, hsa_miR_199b, am-bi_miR_7101, hsa_miR_15b, hsa_miR_371, hsa_miR_151, hsa_miR_19b,, mmu_miR_351, hsa_miR_99b, hsa_miR_508, hsa_miR_140, mmu_miR_346.

Die unterschiedlichen eingesetzten Verfahren bzw. pathophysiologischen Zustände (humane Herzinsuffizienz, Infarktmodell an der Ratte, Hypertrophie-Modell an der Maus durch "aortic banding" nach 3 und 21 Tagen, pharmakologisches in-vitro Modell der kardialen Hypertrophe) führen zu Ergebnissen bei der miRNA-Expression, die gleichen Trends folgen.

Die wirksamsten miRNA-Kandidaten wurden nach den folgenden Kriterien ausgewählt:
(1) Die Auswahl der induzierten miRNAs nach chronischem Myokardinfarkt erfolgte aufgrund der Höhe der Induktion nach Myokardinfarkt (größer ca. 2-fach induziert), als auch aufgrund der zu erwartenden regulierten Zielgene der jeweiligen miRNA (z.B. miRNA-212).
(2) Die Auswahl der induzierten miRNAs in der Herzhypertrophie und Herzinsuffizienz erfolgte nach folgenden Kriterien.
   (a) größer ca. 1,2-fache Induktion in den humanen Herzen von Patienten mit Herzinsuffizienz, und
   (b) mindestens eine weitere Induktion in einem der zusätzlich verwendeten Modelle (Aortic banding nach 3d sowie 21 d; pharmakologisches in vitro Hypertrophie-Modell).

In der folgenden Tabelle 2 sind die SEQ ID NRs, die Bezeichnungen aus der Datenbank (Sanger) und die MI Bezeichnungen zusammengefasst.

**Tabelle 2:**

| **SEQ ID NR.** | **Bezeichnung (Sanger)** | **MI-Bezeichnung** |
|---|---|---|
| SEQ ID NR: 1 | miR-15b | MI 0000438 |
| SEQ ID NR: 2 | miR-23a | MI 0000079 |
| SEQ ID NR: 3 | miR-126* | MI 0000471 |
| SEQ ID NR: 4 | miR-128a | MI 0000155 |
| SEQ ID NR: 5 | miR-134 | MI 0000474 |
| SEQ ID NR: 6 | miR-149 | MI 0000478 |
| SEQ ID NR: 7 | miR-151 | MI 0000809 |
| SEQ ID NR: 8 | miR-206 | MI 0000490 |
| SEQ ID NR: 9 | miR-211 | MI 0000287 |
| SEQ ID NR: 10 | miR-212 | MI 0000288 |
| SEQ ID NR: 11 | miR-214 | MI 0000290 |
| SEQ ID NR: 12 | miR-328 | MI 0000804 |
| SEQ ID NR: 13 | miR-371 | MI 0000779 |
| SEQ ID NR: 14 | miR-21 (hsr miR-21) | MI 0000077 |
| SEQ ID NR: 15 | miR-29b-1 | MI 00000105 |
| SEQ ID NR: 16 | miR-129 | MI 0000252 |
| SEQ ID NR: 17 | miR-213 | MI 0000289 |
| SEQ ID NR: 18 | let-7c | MI 0000064 |
| SEQ ID NR: 19 | miR-106b | MI 0000734 |
| SEQ ID NR: 20 | miR-182 | MI 0000272 |
| SEQ ID NR: 21 | miR-296 | MI 0000747 |
| SEQ ID NR: 22 | miR-122a | MI 0000442 |
| SEQ ID NR: 23 | miR-30a-3p | MI 0000088 |
| SEQ ID NR: 24 | miR-290 | MI 0000388 |
| SEQ ID NR: 25 | miR-30a_5p | MI 0000088 |
| SEQ ID NR: 26 | miR-219 | MI 0000296 |
| SEQ ID NR: 27 | miR-515_5p | MI 0003144 |
| SEQ ID NR: 28 | miR-526b | MI 0003150 |
| SEQ ID NR: 29 | miR-30b | MI 0000441 |

Die bevorzugtesten miRNAs sind nochmals im Folgenden zusammengestellt:

### Hochregulierte miRNAs

(1) miR-212 (SEQ ID NR: 10)
   Diese miRNA ist besonders in der humanen Herzinsuffizienz (5,6-fach) und nach chronischem Myokardinfarkt (2,4-fach) bei der Ratte hochreguliert.
(2) miR-214 (SEQ ID NR: 11)
   Diese miRNA ist leicht in der humanen Herzinsuffizienz (1,2-fach) und deutlich früh und spät nach Herzhypertrophie (aortic banding 3 und 21 Tage) (1,5-fach, bzw. 2,1-fach) bei der Maus sowie nach chronischem Myokardinfarkt (2,6-fach) und nach pharmakologischer Herzhypertrophieinduktion in vitro (1,5-fach) hochreguliert.
(3) miR-21 (SEQ ID NR: 14)
   Diese miRNA ist besonders in der humanen Herzinsuffizienz (3,3-fach) und früh nach Herzhypertrophie (aortic banding 3 Tage) (3,0-fach) bei der Maus hochreguliert.
(4) miR-134 (SEQ ID NR: 5)
   Diese miRNA ist leicht in der humanen Herzinsuffizienz (1,2-fach) und deutlich spät nach Herzhypertrophie (aortic banding 21 Tage) (1,7-fach) bei der Maus sowie nach chronischem Myokardinfarkt (3,3-fach) bei der Ratte hochreguliert.
(5) miR-129 (SEQ ID NR: 16)
   Diese miRNA ist in der humanen Herzinsuffizienz (2,7-fach) und spät nach Herzhypertrophie (aortic banding 21 Tage) (1,4-fach) bei der Maus sowie nach chronischem Myokardinfarkt (1,4-fach) bei der Ratte hochreguliert.

### Herunterregulierte miRNAs

(6) miR-182 (SEQ ID NR: 20)
   Diese miRNA ist in der humanen Herzinsuffizienz (-1,3-fach) und deutlich früh und spät nach Herzhypertrophie (aortic banding 3 und 21 Tage) (-1,8-fach, bzw. -2,2-fach) bei der Maus sowie nach chronischem Myokardinfarkt (-4,0-fach) der der Ratte herunterreguliert.
(7) miR-290 (SEQ ID NR: 24)
   Diese miRNA ist in der humanen Herzinsuffizienz (-1,4-fach) und früh nach Herzhypertrophie (aortic banding 3 Tage) (-1,6-fach) bei der Maus sowie nach chronischem Myokardinfarkt (-1,4-fach) bei der Ratte herunterreguliert.

### Mir-21 als besonders geeignetes therapeutisches Target

Nach Hypertrophieinduktion am Mausmodell fanden die Erfinder eine erhöhte Expression der miR-21 (siehe oben). Sie untersuchten daher ob eine Hemmung der miR-21 *in vivo* zu einer Verhinderung der Entwicklung einer Herzhypertrophie durch Druckbelastung (Verkleinerung des Aortendurchmessers durch aortic banding-Operation) führt. Sie verwendeten zunächst bioinformatorische Analysen und nutzten die Datenbank miRBase (http://microrna.sanger.ac.uk/), um nach potenziellen Targets mit Bindungsstellen für die miR-21 in ihren 3' nichttranslatierten Bereichen zu suchen. Hier identifizierten sie sprouty-1 (SPRY1; Zugangsnummer: NM_001097524), Sarcalumenin (Zugangsnummer: NM_001098814) und Tropomyosin (Zugangsnummer: anschließend NM 001018005) als potenzielle miR-21-Targets. Insbesondere der 3' nichttranslatierte Bereich der SPRY1-mRNA enthält mehrere konservierte miR-Bindungsstellen, von denen eine eine Bindungsstelle für das in der Herzhypertrophie und in der Herzinsuffizienz im Herzen hochregulierte miR-21 ist. Um eine direkte Rolle der miR-21 in der post-transkriptionellen Regulation von SPRY1 und Sarcalumenin zu untersuchen, transfizierten die Erfinder pre-miR-21 Moleküle (Ambion, pre-miRs, 50 nM, 72 h) in kultivierte Kardiomyozyten und analysierten die SPRY1- und Sarcalumenin-Proteinexpression. Sie beobachteten eine starke Reduktion der Proteinexpression von SPRY1 und Sarcalumenin nach pre-miR21 Überexpression in kultivierten Kardiomyozyten (Abb. 7A, B). Eine Reduktion dieser Proteine führt sehr wahrscheinlich zu der Entwicklung der Herzhypertrophie und kardialen Dysfunktion nach Druckbelastung des linken Ventrikels. Die Erfinder beobachteten außerdem eine reduzierte Proteinexpression von SPRY1 (Abb. 7C) und eine verstärkte Phosphorylierung der extrazellulär Signal-regulierten Kinase 1/2 (extracellular signal-regulated kinase 1/2 = phospho-ERK1/2) nach Druckbelastung im linken Ventrikel der Maus (Abb. 7D).

Die Erfinder injizierten anschließend über einen in die Vena jugularis eingebrachten Katheter chemisch modifizierte Oligonukleotide für eine spezifische Herunterregulation der endogenen miR-21 Expression (Antagomir®-21, Alnylam). Die Antagomir® Sequenzen wurden als revers komplementäre Oligonukleotide, wie in der Literatur beschrieben, synthetisiert, wobei Antagomir®-181a zusätzlich mit Cy3 farbmarkiert war (Krutzfeldt et al., 2005).

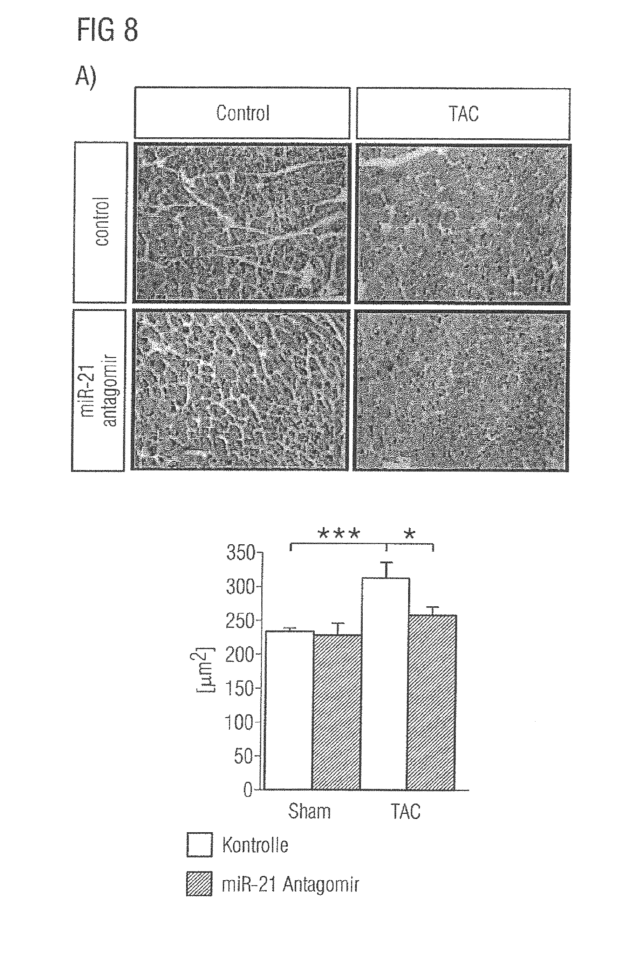

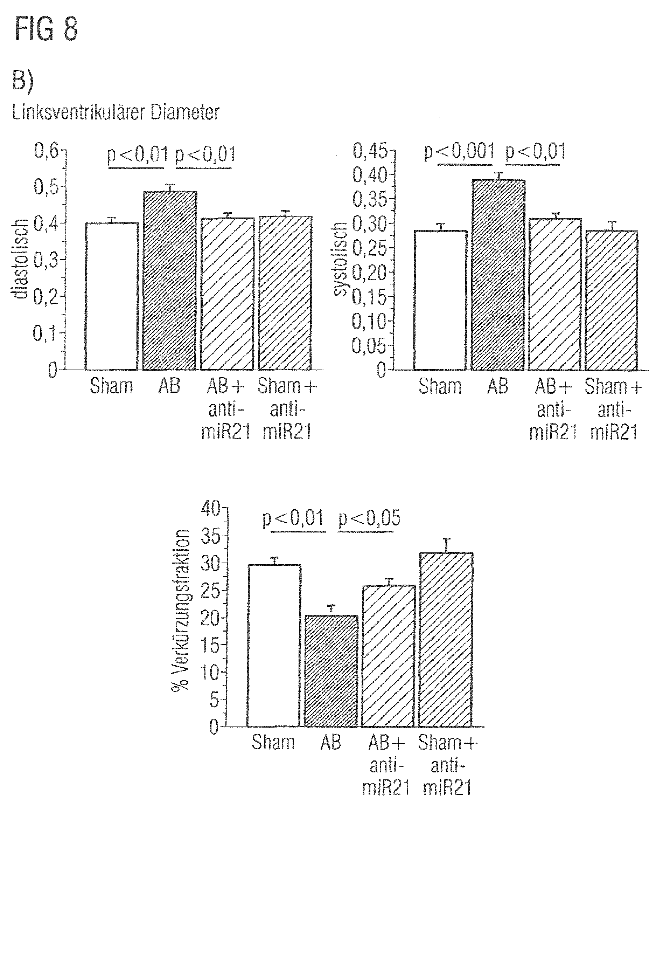

Die Behandlung wurde 24 h nach der operativen Verkleinerung des Aortendurchmessers (aortic banding) begonnen. Es wurden dann jeweils morgens 80 mg/kg/d für 3 Tage 1x pro Tag intravenös appliziert. Nach Injektion eines Cy-3 markierten Antagomir® wurde nach 3 h das Herz entnommen und die kardiale Aufnahme des C3-markierten Antagomir® mittels Immunfluoreszenz analysiert. Es konnte eine starke Aufnahme des Antagomir® in das Herz nachgewiesen werden (Abb. 7E). Echtzeit (real-time) PCR Analysen zeigten überraschenderweise noch nach 3 Wochen eine starke Repression der kardialen miR-21-Expression nach anfänglich 3-tägiger Antagomir®-21-Behandlung (Abb. 7F). Diese Behandlung führte neben der Repression der kardialen miR-21-Expression zu einem Anstieg der SPRY1 Proteinexpression nach aortic banding-Operation (Abb. 7C). Der nach aortic banding beobachtete Anstieg der Expression von phospho-ERK1/2 wurde durch die 3-tägige Antagomir®-21-Behandlung komplett verhindert (Abb. 7D). Das auf das Körpergewicht normalisierte Herzgewicht stieg 3 Wochen nach aortic banding signifikant an und wurde aber durch die Antagomir®-21 Behandlung vollständig normalisiert (Abb. 7G). Die Zunahme der Myozytengröße in Schnitten des Herzens 3 Wochen nach aortic banding-Operation konnte durch Antagomir®-21-Behandlung komplett gehemmt werden (Abb. 8A). Eine Antagomir®-21-Behandlung in Shamoperierten Mäusen führte nicht zu einer Veränderung des Herzgewichts oder der Myozytengröße (Abb. 7G, 8A). Die Herzfunktion wurde mittels Echokardiographie und Katheter-basierten Volumen/Druckmessungen analysiert. Sowohl der linksventrikuläre end-systolische und end-diastolische Diameter erhöhte sich 3 Wochen nach aortic banding, wohingegen das Fractional Shorting (ein Maß für die Herzfunktion) deutlich eingeschränkt war (Abb. 8B). Die durch aortic banding hervorgerufene Druckbelastung des linken Ventrikels führte zu einem messbaren Anstieg des linksventrikulären end-diastolischen Druckes (LVEDp; 2,8±1,3 mmHg (Sham) versus 7,2±1,7mmHg (aortic banding); p=0.09) und zu einer reduzierten kardialen Ejektionsfraktion (55,9%±8,0% (Sham) versus 24,7%±5,6% (aortic banding); p<0.05). Die Antagomir®-21-Behandlung nach aortic banding verhinderte überraschend die linksventrikuläre Dilatation und normalisierte das Fractional Shortening (Abb. 8B). Ebenfalls waren nach Antagomir®-21-Behandlung die Ejektionsfraktion verbessert und der linksventrikuläre end-diastolische Druck normalisiert (EF: 50,1 %±13,1 %; LVEDp: 2,4±1,5 mmHg).

### Ausführungsbeispiele am Menschen - Therapie

Eine Modulation von miRNAs im kardiovaskulären System könnte auch beim Menschen zur Verhinderung von oder als Therapie für kardiovaskuläre Erkrankungen, wie Herzhypertrophie, Herzinsuffizienz oder Myokardinfarkt erfolgreich genutzt werden. Substanzen, welche die miRNA-Expression im kardiovaskulären System erhöhen oder verhindern können über verschiedene Wege appliziert werden wie beispielsweise intravenös, intraarteriell, intrakardial über Katheter oder während einer Operation am offenen Herzen, subkutan, transdermal, inhalativ, oral, rektal, etc. Die Therapie könnte bei Patienten mit Herzhypertrophie, nach Myokardinfarkt oder bei Patienten mit akuter oder chronischer Herzinsuffizienz oder koronarer Herzerkrankung durchgeführt werden. In Anbetracht der bisherigen experimentellen *in vitro* und Tierstudien der Erfinder scheint die Verhinderung der Expression von miR-21, miR-129 und miR-212 erfolgversprechend als kausaler therapeutischer Ansatz zur Verhinderung verschiedener kardiovaskulärer Erkrankungen.

### Ausführungsbeispiele am Menschen - Diagnose

Die Daten belegen, dass eine Änderung der kardialen Expression von verschiedenen miRNAs der Entwicklung einer Herzerkrankung, insbesondere Herzhypertrophie und Herzinsuffizienz, vorausgeht. Um die Expression von miRNAs im Herzen von Patienten zu bestimmen, kann eine Myokardbiopsie entnommen werden. Hieraus kann die miRNA isoliert und die Expressionsstärke bestimmt werden. Eine Erhöhung einzelner miRNAs oder deren Kombination, insbesondere miR-21 (SEQ ID NR: 14), miR-129, miR-212 (SEQ ID NR: 10), miR-214 (SEQ ID NR: 11), miR-134 (SEQ ID NR: 5) kann zur Diagnosestellung eines erhöhten Risikos der Entwicklung oder bereits des Vorliegens einer Herzhypertrophie und/oder Herzinsuffizienz genutzt werden. Eine Herunterregulation der miR-182 (SEQ ID NR: 20) oder miR-290 (SEQ ID NR: 24) oder deren Kombination kann zur Diagnosestellung eines erhöhten Risikos der Entwicklung oder bereits des Vorliegens einer Herzhypertrophie und/oder Herzinsuffizienz genutzt werden. Ebenso kann die Kombination einer erhöhten Expression von miR-21 (SEQ ID NR: 14), miR-129, miR-212 (SEQ ID NR: 10), miR-214 (SEQ ID NR: 11), miR-134 (SEQ ID NR: 5) mit einer erniedrigten Expression von miR-182 (SEQ ID NR: 20) oder miR-290 (SEQ ID NR: 24) zur Diagnosestellung eines erhöhten Risikos der Entwicklung oder bereits des Vorliegens einer Herzhypertrophie und/oder Herzinsuffizienz genutzt werden. Die Expressionsstärke der jeweiligen miRNAs kann entweder über Microarray-Analysen, RT-PCR, Northern Blotting, oder andere geeignete Verfahren erfolgen.

### Referenzen

Buitrago M, Lorenz K, Maass AH, Oberdorf-Maass S, Keller U, Schmitteckert EM, Ivashchenko Y, Lohse MJ, Engelhardt S. The transcriptional repressor Nab1 is a specific regulator of pathological cardiac hypertrophy. Nat Med. 2005 Aug; 11 (8):837-44.
Burkard N, Becher J, Heindl C, Neyses L, Schuh K, Ritter O. (2005) Targeted proteolysis sustains calcineurin activation. Circulation 111:1045-53
Harfe, B. D. (2005). MicroRNAs in vertebrate development. Curr Opin Genet Dev 15, 410-415.
Hornstein, E., Mansfield, J. H., Yekta, S., Hu, J. K.-H., Harfe, B. D., McManus, M. T., Baskerville, S., Bartel, D. P., and Tabin, C. J. (2005). The microRNA miR-196 acts upstream of Hoxb8 and Shh in limb development. Nature 438, 671-674.
Huber W, von Heydebreck A, Sueltmann H, Poustka A, Vingron M: Variance stabilization applied to microarray data calibration and to the quantification of differential expression. Bioinformatics 2002, 18:96-104
Hunter JJ, G. A., Chien KR (1999). Molecular and cellular biology of cardiac hypertrophy and failure (Philadelphia: W. B. Saunders).
Hutvagner, G., and Zamore, P. D. (2002). A microRNA in a multiple-turnover RNAi enzyme complex. Science 297, 2056-2060.
Krutzfeldt J, Rajewsky N, Braich R, Rajeev KG, Tuschl T, Manoharan M, Stoffel M. (2005) Silencing of microRNAs in vivo with 'antagomirs'. Nature 438:685-9.
Lu, J., Getz, G., Miska, E. A., Alvarez-Saavedra, E., Lamb, J., Peck, D., Sweet-Cordero, A., Ebert, B. L., Mak, R. H., Ferrando, A. A., et al. (2005). MicroRNA expression profiles classify human cancers. Nature 435, 834-838.
Massie, B. M., and Shah, N. B. (1997). Evolving trends in the epidemiologic factors of heart failure: rationale for preventive strategies and comprehensive disease management. Am Heart J 133, 703-712.
Meister, G., Landthaler, M., Peters, L., Chen, P. Y., Urlaub, H., Luhrmann, R., and Tuschl, T. (2005). Identification of Novel Argonaute-Associated Proteins. Curr Biol.
Pfeffer, M. A., and Braunwald, E. (1990). Ventricular remodeling after myocardial infarction. Experimental observations and clinical implications. Circulation 81, 1161-1172.
Smyth GK: Linear models and empirical Bayes methods for assessing differential expression in Microarray experiments. Statistical Applications in Molecular Biology, 2004, Vol. 3, No. 1, Article 3
Thum T, Galuppo P, Wolf C, Fiedler J, Kneitz S, van Laake LW, Doevendans PA, Mummery CL, Borlak J, Haverich A, Gross C, Engelhardt S, Ertl G, Bauersachs J. (2007) MicroRNAs in the human heart: a clue to fetal gene reprogramming in heart failure. Circulation 116:258-67.
Thum T, Borlak J. (2001) Reprogramming of gene expression in cultured cardiomyocytes and in explanted hearts by the myosin ATPase inhibitor butanedione monoxime. Transplantation 71:543-52
Thum T, Fraccarollo D, Galuppo P, Tsikas D, Frantz S, Ertl G, Bauersachs J.(2006) Bone marrow molecular alterations after myocardial infarction: Impact on endothelial progenitor cells. Cardiovasc Res 70:50-60
Zhao, Y., Samal, E., and Srivastava, D. (2005). Serum response factor regulates a muscle-specific microRNA that targets Hand2 during cardiogenesis. Nature 436, 214-220.

### Ausführungsformen

1. SEQ ID NR: 1 bis SEQ ID NR: 29 zur Diagnose, Prophylaxe und/oder Therapie von Herzerkrankungen.
2. Verwendung von SEQ ID NR: 1 bis SEQ ID NR: 29 zur Herstellung eines Arzneimittels zur Prophylaxe und/oder Therapie von Herzerkrankungen.
3. Verwendung von SEQ ID NR: 1 bis SEQ ID NR: 29 zur Diagnose von Herzerkrankungen.
4. Verwendung nach Ausführungsform 2 oder 3, wobei die Herzerkrankung Myokardinfarkt, Herzinsuffizienz, chronische Herzinsuffizienz und/oder Herzhypertrophie ist.
5. Verfahren zur Diagnose einer Herzerkrankung, wobei das Verfahren die Schritte umfasst:
   (a) Bereitstellen einer Probe eines Individuums, das unter dem Verdacht einer Herzerkrankung steht;
   (b) Messen der Expressionsmenge von mindestens einer Sequenz ausgewählt unter SEQ ID NR: 1 bis SEQ ID NR: 29 in der Probe;
   wobei eine erhöhte oder verringerte Expressionsmenge mindestens einer Sequenz ausgewählt unter SEQ ID NR: 1 bis SEQ ID NR: 29 gegenüber einer Kontrollprobe eine Herzerkrankung oder eine Prädisposition für eine Herzerkrankung anzeigt.
6. Verfahren nach Ausführungsform 5, wobei die Sequenz SEQ ID NR: 5 (miR-134), SEQ ID NR: 10 (miR-212), SEQ ID NR: 11 (miR-214), SEQ ID NR: 14 (miR- 21), miR-129 (SEQ ID NR: 16), SEQ ID NR: 20 (miR-182) und/oder SEQ ID NR: 24 (miR-290) ist.
7. Kit umfassend mindestens eine Sequenz ausgewählt unter SEQ ID NR: 1 bis SEQ ID NR: 29 zur Diagnose von Herzerkrankungen.
8. Expressionsvektor umfassend mindestens eine Sequenz ausgewählt unter SEQ ID NR: 1 bis SEQ ID NR: 29.
9. Zelle umfassend mindestens einen Expressionsvektor nach Ausführungsform 8.
10. Verfahren zum Modulieren einer Herzerkrankung umfassend das Modulieren der Expression oder Aktivität mindestens einer Sequenz, die ausgewählt ist unter SEQ ID NR: 1 bis SEQ ID NR: 29 in einer Zelle.
11. Verfahren zum Screenen einer pharmazeutisch aktiven Verbindung zur Behandlung und/oder Prophylaxe einer Herzerkrankung, wobei das Verfahren die Schritte umfasst:
   (a) Bereitstellen einer Zelle nach Ausführungsform 9,
   (b) In Kontakt bringen eines Kandidaten einer pharmazeutisch aktiven Verbindung mit der Zelle,
   (c) Bestimmen der Wirkung des Kandidaten auf die Expression einer Sequenz, die ausgewählt ist unter SEQ ID NR: 1 bis SEQ ID NR: 29, wobei eine Veränderung der Expression mindestens einer Sequenz von SEQ ID NR: 1 bis SEQ ID NR: 29 eine pharmazeutisch aktive Verbindung anzeigt.

## Patentansprüche

1. SEQ ID NR: 14 zur Diagnose, Prophylaxe und/oder Therapie von Herzerkrankungen.

2. Verwendung von SEQ ID NR: 14 zur Herstellung eines Arzneimittels zur Prophylaxe und/oder Therapie von Herzerkrankungen.

3. Verwendung von SEQ ID NR: 14 zur Diagnose von Herzerkrankungen.

4. Verwendung nach Anspruch 2 oder 3, wobei die Herzerkrankung Myokardinfarkt, Herzinsuffizienz, chronische Herzinsuffizienz und/oder Herzhypertrophie ist.

5. Verfahren zur Diagnose einer Herzerkrankung, wobei das Verfahren die Schritte umfasst:
(a) Bereitstellen einer Probe eines Individuums, das unter dem Verdacht einer Herzerkrankung steht;
(b) Messen der Expressionsmenge der Sequenz SEQ ID NR: 14 in der Probe;
wobei eine erhöhte oder verringerte Expressionsmenge von SEQ ID NR: 14 gegenüber einer Kontrollprobe eine Herzerkrankung oder eine Prädisposition für eine Herzerkrankung anzeigt.

6. Kit umfassend mindestens die Sequenz SEQ ID NR: 14 zur Diagnose von Herzerkrankungen.

7. Expressionsvektor umfassend die Sequenz SEQ ID NR: 14.

8. Zelle umfassend mindestens einen Expressionsvektor nach Anspruch 8.

9. Verfahren zum Modulieren einer Herzerkrankung umfassend das Modulieren der Expression oder Aktivität der Sequenz SEQ ID NR: 14 in einer Zelle.

10. Verfahren zum Screenen einer pharmazeutisch aktiven Verbindung zur Behandlung und/oder Prophylaxe einer Herzerkrankung, wobei das Verfahren die Schritte umfasst:
(a) Bereitstellen einer Zelle nach Anspruch 8,
(b) In Kontakt bringen eines Kandidaten einer pharmazeutisch aktiven Verbindung mit der Zelle,
(c) Bestimmen der Wirkung des Kandidaten auf die Expression der Sequenz SEQ ID NR: 14,
wobei eine Veränderung der Expression der Sequenz SEQ ID NR: 14eine pharmazeutisch aktive Verbindung anzeigt.


SEQUENCE LISTING

<110> Julius-Maximilians-Universitaet Wuerzburg

<120> Micro RNA (miRNA) zur Diagnose und Therapie von Herzerkrankungen

<130> P059194EP

<150> EP 06090187.3
<151> 2006-10-09

<150> PCT/EP2007/008772
<151> 2007-10-09

<150> EP 07818845.5
<151> 2007-10-09

<150> EP 10161572.2
<151> 2007-10-09

<160> 29

<170> PatentIn version 3.5

<210> 1
<211> 22
<212> RNA
<213> Homo sapiens

<400> 1
uagcagcaca ucaugguuua ca 22

<210> 2
<211> 21
<212> RNA
<213> Homo sapiens

<400> 2
aucacauugc cagggauuuc c 21

<210> 3
<211> 21
<212> RNA
<213> Homo sapiens

<400> 3
cauuauuacu uuugguacgc g 21

<210> 4
<211> 22
<212> RNA
<213> Homo sapiens

<400> 4
ucacagugaa ccggucucuu uu 22

<210> 5
<211> 21
<212> RNA
<213> Homo sapiens

<400> 5
ugugacuggu ugaccagagg g                                                21

<210> 6
<211> 22
<212> RNA
<213> Homo sapiens

<400> 6
ucuggcuccg ugucuucacu cc                                               22

<210> 7
<211> 22
<212> RNA
<213> Homo sapiens

<400> 7
acuagacuga agcuccuuga gg                                               22

<210> 8
<211> 22
<212> RNA
<213> Homo sapiens

<400> 8
uggaauguaa ggaagugugu gg                                               22

<210> 9
<211> 22
<212> RNA
<213> Homo sapiens

<400> 9
uucccuuugu cauccuucgc cu                                               22

<210> 10
<211> 21
<212> RNA
<213> Homo sapiens

<400> 10
uaacagucuc cagucacggc c                                                21

<210> 11
<211> 21
<212> RNA
<213> Homo sapiens

<400> 11
acagcaggca cagacaggca g                                                21

<210> 12
<211> 22
<212> RNA
<213> Homo sapiens

<400> 12
cuggcccucu cugcccuucc gu                                               22

<210> 13
<211> 21
<212> RNA
<213> Homo sapiens

<400> 13
gugccgccau cuuuugagug u                                                21


<210> 14
<211> 22
<212> RNA
<213> Homo sapiens

<400> 14
uagcuuauca gacugauguu ga                                               22


<210> 15
<211> 23
<212> RNA
<213> Homo sapiens

<400> 15
uagcaccauu ugaaaucagu guu                                              23


<210> 16
<211> 21
<212> RNA
<213> Homo sapiens

<400> 16
cuuuuugcgg ucugggcuug c                                                21


<210> 17
<211> 23
<212> RNA
<213> Homo sapiens

<400> 17
aacauucaac gcugucggug agu                                              23


<210> 18
<211> 22
<212> RNA
<213> Homo sapiens

<400> 18
ugagguagua gguuguaugg uu                                               22


<210> 19
<211> 21
<212> RNA
<213> Homo sapiens

<400> 19
uaaagugcug acagugcaga u                                                21

<210> 20
<211> 22
<212> RNA
<213> Homo sapiens

<400> 20
uuuggcaaug guagaacuca ca                                                22


<210> 21
<211> 21
<212> RNA
<213> Homo sapiens

<400> 21
agggcccccc cucaauccug u                                                 21


<210> 22
<211> 23
<212> RNA
<213> Homo sapiens

<400> 22
uggaguguga caaugguguu ugu                                               23


<210> 23
<211> 22
<212> RNA
<213> Homo sapiens

<400> 23
uguaaacauc cucgacugga ag                                                22


<210> 24
<211> 23
<212> RNA
<213> Homo sapiens

<400> 24
cucaaacuau gggggcacuu uuu                                               23


<210> 25
<211> 22
<212> RNA
<213> Homo sapiens

<400> 25
uguaaacauc cucgacugga ag                                                22


<210> 26
<211> 21
<212> RNA
<213> Homo sapiens

<400> 26
ugauugucca aacgcaauuc u                                                 21


<210> 27
<211> 24

<212> RNA
<213> Homo sapiens

<400> 27
uucuccaaaa gaaagcacuu ucug                                              24

<210> 28
<211> 24
<212> RNA
<213> Homo sapiens

<400> 28
cucuugaggg aagcacuuuc uguu                                              24

<210> 29
<211> 22
<212> RNA
<213> Homo sapiens

<400> 29
uguaaacauc cuacacucag cu                                                22